# EUROPEAN PATENT SPECIFICATION

(11) **EP 1 061 946 B1**
(45) Date of publication and mention of the grant of the patent: **28.04.2004**
(21) Application number: 99906077.5
(22) Date of filing: 25.02.1999
(51) Int. Cl.: A61K 38/26, C07K 14/605

(54) **GLP-1 DERIVATIVES WITH HELIX-CONTENT EXCEEDING 25 %, FORMING PARTIALLY STRUCTURED MICELLAR-LIKE AGGREGATES**
GLP-1 DERIVATE MIT EINEM HELIX-GEHALT ÜBER 25 %, DIE PARTIELL STRUKTURIERTE MIZELLENARTIGE AGGREGATE BILDEN
DERIVES DE GLP-1 AVEC UNE TENEUR DE STRUCTURES EN HELICES SUPERIEURE A 25 %, FORMANT DES AGREGATS DE TYPE MICELLAIRE PARTIELLEMENT STRUCTURES

(30) Priority: 27.02.1998 DK 26898; 27.02.1998 DK 27298
(43) Date of publication of application: 27.12.2000
(73) Proprietor: NOVO NORDISK A/S, 2880 Bagsvaerd (DK)
(72) Inventor: KNUDSEN, Liselotte, Bjerre, DK-2500 Valby (DK); HUUSFELDT, Per, Olaf, DK-2100 Copenhagen OE (DK); NIELSEN, Per, Franklin, DK-3500 V rl se (DK); KAARSHOLM, Niels, C., DK-2720 Vanl se (DK); OLSEN, Helle, Birk, DK-3450 Aller d (DK); BJORN, Soren, Erik, DK-2800 Lyngby (DK)
(74) Representative: Kjerrumgaard, Lars
(86) International application number: PCT/DK1999/000084
(87) International publication number: WO 1999/043341

(56) References cited:
- WO-A1-87/06941
- WO-A1-90/11296
- WO-A1-91/11457
- WO-A1-98/08871
- US-A- 5 614 492
- JOURNAL OF PHARMACEUTICAL SCIENCES, Volume 89, No. 8, August 1994, YESOOK KIM et al., "FT-IR and Near-Infared FT-Raman Studies of the Secondary Structure of Insulinotropin in the Solid State; alpha-Helix to beta-Sheet Concersion Induced by Phenol and/or by High Shear Force", pages 1173-1180.
- PHARMACEUTICAL RESEARCH, Volume 15, No. 2, 1998, DEAN K. CLODFELTER et al., "Effects of Non-Covalent Self-Association on the Subcutaneous Absorption of a Therapeutic Peptide", pages 254-262.

## Description

### Field of the invention

The present invention relates to a pharmaceutical composition comprising a GLP-1 derivative of improved solubility and/or stability, and to a method for improving the solubility and/or stability of GLP-1 or a fragment or an analogue thereof.

### Background of the invention

Peptides are widely used in medical practice, and since they can be produced by recombinant DNA technology it can be expected that their importance will increase also in the years to come.

The hormones regulating insulin secretion belong to the so-called enteroinsular axis, designating a group of hormones, released from the gastrointestinal mucosa in response to the presence and absorption of nutrients in the gut, which promote an early and potentiated release of insulin. The enhancing effect on insulin secretion, the so-called incretin effect, is probably essential for a normal glucose tolerance. Many of the gastrointestinal hormones, including gastrin and secretin (cholecystokinin is not insulinotropic in man), are insulinotropic, but the only physiologically important ones, those that are responsible for the incretin effect, are the glucose-dependent insulinotropic polypeptide, GIP, and glucagon-like peptide-1 (GLP-1). Because of its insulinotropic effect, GIP, isolated in 1973 (1) immediately attracted considerable interest among diabetologists. However, numerous investigations carried out during the following years clearly indicated that a defective secretion of GIP was not involved in the pathogenesis of insulin dependent diabetes mellitus (IDDM) or non insulin-dependent diabetes mellitus (NIDDM) (2). Furthermore, as an insulinotropic hormone, GIP was found to be almost ineffective in NIDDM (2). The other incretin hormone, GLP-1 is the most potent insulinotropic substance known (3). Unlike GIP, it is surprisingly effective in stimulating insulin secretion in NIDDM patients. In addition, and in contrast to the other insulinotropic hormones (perhaps with the exception of secretin) it also potently inhibits glucagon secretion. Because of these actions it has pronounced blood glucose lowering effects particularly in patients with NIDDM.

GLP-1, a product of the proglucagon (4), is one of the youngest members of the secretin-VIP family of peptides, but is already established as an important gut hormone with regulatory function in glucose metabolism and gastrointestinal secretion and metabolism (5). The glucagon gene is processed differently in the pancreas and in the intestine. In the pancreas (9), the processing leads to the formation and parallel secretion of 1) glucagon itself, occupying positions 33-61 of proglucagon (PG); 2) an N-terminal peptide of 30 amino acids (PG (1-30)) often called glicentin-related pancreatic peptide, GRPP (10, 11); 3) a hexapeptide corresponding to PG (64-69); 4) and, finally, the so-called major proglucagon fragment (PG (72-158)), in which the two glucagon-like sequences are buried (9). Glucagon seems to be the only biologically active product. In contrast, in the intestinal mucosa, it is glucagon that is buried in a larger molecule, while the two glucagon-like peptides are formed separately (8). The following products are formed and secreted in parallel: 1) glicentin, corresponding to PG (1-69), with the glucagon sequence occupying residues Nos. 33-61 (12); 2) GLP-1(7-36)amide (PG (78-107))amide (13), not as originally believed PG (72-107)amide or 108, which is inactive). Small amounts of C-terminally glycine-extended but equally bioactive GLP-1(7-37), (PG (78-108)) are also formed (14); 3) intervening peptide-2 (PG (111-122)amide) (15); and 4) GLP-2 (PG (126-158)) (15, 16). A fraction of glicentin is cleaved further into GRPP (PG (1-30)) and oxyntomodulin (PG (33-69)) (17, 18). Of these peptides, GLP-1, has the most conspicuous biological activities.

The amino acid sequence of GLP-1 is given *i.a.* by Schmidt *et al*. (*Diabetologia* **28** 704-707 (1985). Although the interesting pharmacological properties of GLP-1 (7-37) and analogues thereof have attracted much attention in recent years only little is known about the structure of these molecules. The secondary structure of GLP-1 in micelles has been described by Thorton *et al*. (*Biochemistry* **33** 3532-3539 (1994)), but in normal solution, GLP-1 is considered a very flexible molecule. Surprisingly, we found that derivatisation of this relatively small and very flexible molecule resulted in compounds whose plasma profile were highly protracted and still had retained activity (PCT application No. DK97/00340).

While much attention has been focused on the pharmacological properties of acylated GLP-1 derivatives, hitherto little is known about their physico-chemical and solution structural properties. Such knowledge is a prerequisite for rational handling during e.g. production, purification and formulation work and is eventually important for understanding of the structural basis for the protraction mechanism.

GLP-1 and analogues of GLP-1 and fragments thereof are potentially useful *i*.*a*. in the treatment of type 1 and type 2 diabetes. However, solubility limitations and the low stability against the actions of endogenous diaminopeptidyl peptidase limits the usefulness of these compounds, and thus there still is a need for improvements in this field. Accordingly, it is one object of the present invention to provide pharmaceutical solutions comprising GLP-1 derivatives with improved solubility and stability.

### References.

1. Pederson RA. Gastric Inhibitory Polypeptide. In Walsh JH, Dockray GJ (eds) Gut peptides: Biochemistry and Physiology. Raven Press, New York 1994, pp. 217259.
2. Krarup T. Immunoreactive gastric inhibitory polypeptide. Endocr Rev 1988;9:122-134.
3. Ørskov C. Glucagon-like peptide-1, a new hormone of the enteroinsular axis. Diabetologia 1992; 35:701-711.
4. Bell Gl, Sanchez-Pescador R, Laybourn PJ, Najarian RC. Exon duplication and divergence in the human preproglucagon gene. Nature 1983; 304: 368-371.
5. Holst JJ. Glucagon-like peptide-1 (GLP-1) - a newly discovered Gl hormone. Gastroenterology 1994; 107: 1848-1855.
6. Holst JJ. Gut glucagon, enteroglucagon, gut GLI, glicentin - current status. Gastroenterology 1983;84:1602-1613.
7. Holst JJ, Ørskov C. Glucagon and other proglucagon-derived peptides. In Walsh JH, Dockray GJ, eds. Gut peptides: Biochemistry and Physiology. Raven Press, New York, pp. 305-340, 1993.
8. Ørskov C, Hoist JJ, Knuhtsen S, Baldissera FGA, Poulsen SS, Nielsen OV. Glucagon-like peptides GLP-1 and GLP-2, predicted products of the glucagon gene, are secreted separately from the pig small intestine, but not pancreas. Endocrinology 1986;119:1467-1475.
9. Holst JJ, Bersani M, Johnsen AH, Kofod H, Hartmann B, Ørskov C. Proglucagon processing in porcine and human pancreas. J Biol Chem, 1994; 269: 18827-1883.
10. Moody AJ, Holst JJ, Thim L, Jensen SL. Relationship of glicentin to proglucagon and glucagon in the porcine pancreas. Nature 1981; 289: 514-516.
11. Thim L, Moody AJ, Purification and chemical characterisation of a glicentin-related pancreatic peptide (proglucagon fragment) from porcine pancreas. Biochim Biophys Acta 1982;703:134-141.
12. Thim L, Moody AJ. The primary structure of glicentin (proglucagon). Regul Pept 1981;2:139-151.
13. Ørskov C, Bersani M, Johnsen AH, Højrup P, Holst JJ. Complete sequences of glucagon-like peptide-1 (GLP-1) from human and pig small intestine. J. Biol. Chem. 1989;264:12826-12829.
14. Ørskov C, Rabenhøj L, Kofod H Wettergren A, Holst JJ. Production and secretion of amidated and glycine-extended glucagon-like peptide-1 (GLP-1) in man. Diabetes 1991; 43: 535-539.
15. Buhl T, Thim L. Kofod H, Ørskov C, Harling H, & Holst JJ: Naturally occurring products of proglucagon 111-160 in the porcine and human small intestine. J. Biol. Chem. 1988;263:8621-8624.
16. Ørskov C, Buhl T, Rabenhøj L, Kofod H, Holst JJ: Carboxypeptidase-B-like processing of the C-terminus of glucagon-like peptide-2 in pig and human small intestine. FEBS letters, 1989;247:193-106.
17. Holst JJ. Evidence that enteroglucagon (II) is identical with the C-terminal sequence (residues 33-69) of glicentin. Biochem J. 1980;187:337-343.
18. Bataille D, Tatemoto K, Gespach C, Jörnvall H, Rosselin G, Mutt V. Isolation of glucagon-37 (bioactive enteroglucagon/oxyntomodulin) from porcine jejune-ileum. Characterisation of the peptide. FEBS Lett 1982;146:79-86.
19. Ørskov C, Wettergren A, Holst JJ. The metabolic rate and the biological effects of GLP-1 7-36amide and GLP-1 7-37 in healthy volunteers are identical. Diabetes 1993;42:658-661.
20. Elliott RM, Morgan LM, Tredger JA, Deacon S, Wright J, Marks V. Glucagon-like peptide-1 (7-36)amide and glucose-dependent insulinotropic polypeptide secretion in response to nutrient ingestion in man: acute post-prandial and 24-h secretion patterns. J Endocrinol 1993; 138: 159-166.
21. Kolligs F, Fehmann HC, Göke R, Göke B. Reduction of the incretin effect in rats by the glucagon-like peptide-1 receptor antagonist exendin (9-39)amide. Diabetes 1995; 44: 16-19.
22. Wang Z, Wang RM, Owji AA, Smith DM, Ghatei M, Bloom SR. Glucagon-like peptide-1 is a physiological incretin in rat. J. Clin. Invest. 1995; 95: 417-421.
23. Thorens B. Expression cloning of the pancreatic b cell receptor for the gluco-incretin hormone glucagon-like peptide 1. Proc Natl Acad Sci 1992;89:8641-4645.
24. Scrocchi L, Auerbach AB, Joyner AL, Drucker DJ. Diabetes in mice with targeted disruption of the GLP-1 receptor gene. Diabetes 1996; 45: 21A.
25. Fehmann HC, Göke R, Göke B. Cell and molecular biology of the incretin hormones glucagon-like peptide-I (GLP-1) and glucose-dependent insulin releasing polypeptide (GIP). Endocrine Reviews, 1995; 16: 390-410.
26. Gromada J, Dissing S, Bokvist K, Renström E, Frøkjær-Jensen J, Wulff BS, Rorsman P. Glucagon-like peptide I increases cytoplasmic calcium in insulin-secreting bTC3-cells by enhancement of intracellular calcium mobilisation. Diabetes 1995; 44: 767-774.
27. Holz GG, Leech CA, Habener JF. Activation of a cAMP-regulated Ca²⁺-signaling pathway in pancreatic β-cells by the insulinotropic hormone glucagon-like peptide-1. J Biol Chem, 1996; 270:17749-17759.
28. Holz GG, Kühltreiber WM, Habener JF. Pancreatic beta-calls are rendered glucose competent by the insulinotropic hormone glucagon-like peptide-1 (7-37). Nature 1993;361:362-365.
29. Ørskov C, Holst JJ, Nielsen OV: Effect of truncated glucagon-like peptide-1 (proglucagon 78-107 amide) on endocrine secretion from pig pancreas, antrum and stomach. Endocrinology 1988;123:2009-2013.
30. Hvidberg A, Toft Nielsen M, Hilsted J, Ørskov C, Holst JJ. Effect of glucagon-like peptide-1 (proglucagon 78-107amide) on hepatic glucose production in healthy man. Metabolism 1994;43:104-108.
31. Qualmann C, Nauck M, Holst JJ, Ørskov C, Creutzfeldt W. Insulinotropic actions of intravenous glucagon-like peptide-1 [7-36 amide] in the fasting state in healthy subjects. Acta Diabetologica, 1995; 32: 13-16.
32. Nauck MA, Heimesaat MM, Ørskov C, Holst JJ, Ebert R, Creutzfeldt W. Preserved incretin activity of GLP-1 (7-36amide) but not of synthetic human GIP in patients with type 2-diabetes mellitus. J Clin Invest 1993;91:301-307.
33. Nauck MA, Kleine N, Ørskov C, Hoist JJ, Willms B, Creutzfeldt W. Normalisation of fasting hyperglycaemia by exogenous GLP-1(7-36amide) in type 2-diabetic patients. Diabetologia 1993;36:741-744.
34. Creutzfeldt W, Kleine N, Willms B, Ørskov C, Holst JJ, Nauck MA. Glucagonostatic actions and reduction of fasting hyperglycaemia by exogenous glucagon-liem, peptide-1 (7-36amide) in type I diabetic patients. Diabetes Care 1996; 19: 580-586.
35. Schjoldager BTG, Mortensen PE, Christiansen J, Ørskov C, Holst JJ. GLP-1 (glucagon-like peptide-1) and truncated GLP-1, fragments of human proglucagon, inhibit gastric acid secretion in man. Dig. Dis. Sci. 1989; 35:703-708.
36. Wettergren A, Schjoldager B, Mortensen PE, Myhre J, Christiansen J, Holst JJ. Truncated GLP-1 (proglucagon 72-107amide) inhibits gastric and pancreatic functions in man. Dig Dis Sci 1993;38:665-673.
37. Layer P, Holst JJ, Grandt D, Goebell H: Ileal release of glucagon-like peptide-1 (GLP-1): association with inhibition of gastric acid in humans. Dig Dis Sci 1995; 40: 1074-1082.
38. Layer P, Holst JJ. GLP-1: A humoral mediator of the ileal brake in humans? Digestion 1993; 54: 385-386.
39. Nauck M, Ettler R, Niedereichholz U, Ørskov C, Holst JJ, Schmiegel W. Inhibition of gastric emptying by GLP-1 (7-36 amide) or (7-37): effects on postprandial glycaemia and insulin secretion. Abstract. Gut 1995; 37 (suppl. 2): A124.
40. Schick RR, vorm Walde T, Zimmermann JP, Schusdziarra V, Classen M. Glucagon-like peptide 1 - a novel brain peptide involved in feeding regulation. in Ditschuneit H, Gries FA, Hauner H, Schusdziarra V, Wechsler JG (eds.) Obesity in Europe. John Libbey & Company Itd, 1994; pp. 363-367.
41. Tang-Christensen M, Larsen PJ, Göke R, Fink-Jensen A, Jessop DS, Møller M, Sheikh S. Brain GLP-1(7-36) amide receptors play a major role in regulation of food and water intake. Am. J. Physiol., 1996, in press.
42. Turton MD, O'Shea D, Gunn I, Beak SA, Edwards CMB, Meeran K, et al. A role for glucagon-like peptide-1 in the regulation of feeding. Nature 1996; 379: 69-72.
43. Willms B, Werner J, Creutzfeldt W, Ørskov C, Holst JJ, Nauck M. Inhibition of gastric emptying by glucagon-like peptide-1 (7-36 amide) in patients with type-2-diabetes mellitus. Diabetologia 1994; 37, suppl. 1: A118.
44. Larsen J, Jallad N, Damsbo P. One-week continuous infusion of GLP-1(7-37) improves glycaemic control in NIDDM. Diabetes 1996; 45, suppl. 2: 233A.
45. Ritzel R, Ørskov C, Hoist JJ, Nauck MA. Pharmacokinetic, insulinotropic, and glucagonostatic properties of GLP-1 [7-36 amide] after subcutaneous injection in healthy volunteers. Dose-response relationships. Diabetologia 1995; 38: 720-725.
46. Deacon CF, Johnsen AH, Hoist JJ. Degradation of glucagon-like peptide-1 by human plasma in vitro yields an N-terminally truncated peptide that is a major endogenous metabolite in vivo. J Clin Endocrinol Metab 1995; 80: 952-957.
47. Deacon CF, Nauck MA, Toft-Nielsen M, Pridal L, Wilims B, Holst JJ. 1995. Both subcutaneous and intravenously administered glucagon-like peptide-1 are rapidly degraded from the amino terminus in type II diabetic patients and in healthy subjects. Diabetes 44: 1126-1131.

### Summary of the invention

Human GLP-1 is a 37 amino acid residue peptide originating from preproglucagon which is synthesised *i*.*a*. in the L-cells in the distal ileum, in the pancreas and in the brain. Processing of preproglucagon to give GLP-1 (7-36)amide, GLP-1 (7-37) and GLP-2 occurs mainly in the L-cells. A simple system is used to describe fragments and analogues of this peptide. Thus, for example, Gly⁸-GLP-1 (7-37) designates a fragment of GLP-1 formally derived from GLP-1 by deleting the amino acid residues Nos. 1 to 6 and substituting the naturally occurring amino acid residue in position 8 (Ala) by Gly. Similarly, Lys³⁴(N^{ε}-tetradecanoyl)-GLP-1(7-37) designates GLP-1(7-37) wherein the ε-amino group of the Lys residue in position 34 has been tetradecanoylated. Where reference in this text is made to C-terminally extended GLP-1 analogues, the amino acid residue in position 38 is Arg unless otherwise indicated, the optional amino add residue in position 39 is also Arg unless otherwise indicated and the optional amino add residue in position 40 is Asp unless otherwise indicated. Also, if a C-terminally extended analogue extends to position 41, 42, 43, 44 or 45, the amino acid sequence of this extension is as in the corresponding sequence in human preproglucagon unless otherwise indicated.

PCT application No. DK97/00340 describes various GLP-1 derivatives that are found to be very protracted. Whereas GLP-1 and GLP-1 analogues are molecules to which no defined solution structure can be ascribed, we found that some of these protracted GLP-1 derivatives may exist in a partially structured micellar-like aggregated form which is stable over a wide concentration range.

Circular Dichroism (CD) can be used to show that the GLP-1 derivatives have a certain partially structured conformation independent of their concentration. In contrast, for normal GLP-1(7-37) an increase in the helix content is seen with increasing concentration, from 10-15% to 30-35% (at 500 µM concentration) in parallel with peptide self-association. For the GLP-1 derivatives forming partially structured micellar-like aggregates in aqueous solution the helix content remains constant above 30% at concentrations of 10 µM. The aggregated structured conformation is an inherent property of the derivative present in water or dilute aqueous buffer without the need for any additional structure-inducing components.

Thus, in its broadest aspect, the present invention relates to a pharmaceutical composition comprising a GLP-1 derivative which has a helix content as measured by CD at 222 nm in H₂O at 22 ± 2 °C exceeding 25%, preferably in the range of 25% to 50%, at a peptide concentration of about 10 µM, wherein the concentration of the GLP-1 derivative is not less than 0,5 mg/ml, and a preservative.

The size of the partially helical, micelle-like aggregates may be estimated by size-exclusion chromatography. Similarly, the apparent (critical micelle concentrations) CMC's of the peptides may be estimated from the concentration dependent fluorescence in the presence of appropriate dyes (e.g. Brito, R. & Vaz, W. (1986) Anal. Biochem. **152,** 250-255).

That the derivatives have a partially structured micellar-like aggregate conformation in aqueous solutions makes them more soluble and stable in solution as compared to the native peptide. The increased solubility and stability can be seen by comparing the solubility after 9 days of standing for a derivative and normal GLP-1(7-37) in a pharmaceutical formulation, e.g. 5 mM phosphate buffer, pH 6.9 added 0.1 M NaCl.

In the present text, the designation "an analogue" is used to designate a peptide wherein one or more amino acid residues of the parent peptide have been substituted by another amino acid residue and/or wherein one or more amino acid residues of the parent peptide have been deleted and/or wherein one or more amino acid residues have been added to the parent peptide. Such addition can take place either at the N-terminal end or at the C-terminal end of the parent peptide or both.

The term "derivative" is used in the present text to designate a peptide in which one or more of the amino acid residues of the parent peptide have been chemically modified, *e.g.* by alkylation, acylation, ester formation or amide formation.

The term "a GLP-1 derivative" is used in the present text to designate a derivative of GLP-1 or an analogue thereof. In the present text, the parent peptide from which such a derivative is formally derived is in some places referred to as the "GLP-1 moiety" of the derivative.

In a preferred embodiment, the present invention relates to pharmaceutical composition according to claim 1, wherein the concentration of GLP-1 derivative is not less than 0.5 mg/ml, preferably not less than about 5 mg/ml, more preferred not less than about 10 mg/ml and, preferably, not more than about 100 mg/ml.

The pharmaceutical composition of the invention preferably comprises a GLP-1 derivative wherein at least one amino acid residue of the parent peptide has a lipophilic substituent attached. More preferred are compositions comprising a GLP-1 derivative having a lipophilic-substituent which is attached to any one of the amino acid residues in position 18-38, preferably 26-34.

The pharmaceutical composition according to the invention, preferably further comprises one or more of the following substances:
- a pharmaceutically acceptable vehicle or carrier;
- an isotonic agent, preferably selected from the group consisting of sodium chloride, mannitol and glycerol;
- a preservative, preferably selected from the group consisting of phenol, m-cresol, methyl p-hydroxybenzoate, butyl p-hydroxybenzoate and benzyl alcohol;
- a buffer, preferably selected from the group consisting of sodium acetate, citrate, glycylglycine, histidine, 2-phenylethanol and sodium phosphate; and
- a surfactant capable of improving the solubility and/or the stability of the GLP-1 derivative, preferable selected from poloxymer 188, tween 20 and tween 80.

In a preferred embodiment, the pharmaceutical composition of the invention comprises a GLP-1 derivative wherein the lipophilic substituent comprises from 4 to 40 carbon atoms, preferably from 8 to 25 carbon atoms.

The lipophilic substituent is preferably attached to an amino acid residue in such a way that a carboxyl group of the lipophilic substituent forms an amide bond with an amino group of the amino add residue, or, the lipophilic substituent is attached to an amino acid residue in such a way that an amino group of the lipophilic substituent forms an amide bond with a carboxyl group of the amino add residue.

In a preferred embodiment the pharmaceutical composition according to the invention comprises a GLP-1 derivative wherein the lipophilic substituent is attached to the parent peptide by means of a spacer.

The spacer is preferably, in one embodiment, an unbranched alkane α,ω-dicarboxylic acid group having from 1 to 7 methylene groups, preferably two methylene groups, which form a bridge between an amino group of the parent peptide and an amino group of the lipophilic substituent.

The spacer is preferably, in another embodiment, an amino add residue except Cys, or a dipeptide such as Gly-Lys or any unbranched alkane α,ω-aminoacid having from 1 to 7 methylene groups, preferably 2-4 methylene groups, which form a bridge between an amino group of the parent peptide and an amino group of the lipophile substituent.

In a preferred embodiment, the lipophilic substituent comprises a partially or completely hydrogenated cyclopentanophenathrene skeleton.

In another preferred embodiment, the lipophilic substituent is a straight-chain or branched alkyl group.

The lipophilic substituent is preferably the acyl group of a straight-chain or branched fatty add, the acyl group more preferably being:
- selected from the group comprising CH₃(CH₂)ₙCO-, wherein n is 4 to 38, preferably CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-; or
- an acyl group of a straight-chain or branched alkane α,ω-dicarboxylic acid; or
- selected from the group comprising HOOC(CH₂)ₘCO-, wherein m is from 4 to 38, preferably from 4 to 24, more preferred selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

In another preferred embodiment, the lipophilic substituent is a group of the formula CH₃(CH₂)ₚ((CH₂)_{q}COOH)CHNH-CO(CH₂)₂CO-, wherein p and q are integers and p+q is an integer of from 8 to 33, preferably from 12 to 28.

In another preferred embodiment, the lipophilic substituent is a group of the formula CH₃(CH₂)ᵣCO-NHCH(COOH)(CH₂)₂CO-, wherein r is an integer of from 10 to 24.

In another preferred embodiment, the lipophilic substituent is a group of the formula CH₃(CH₂)₈CO-NHCH((CH₂)₂COOH)CO-, wherein s is an integer of from 8 to 24.

In another preferred embodiment, the lipophilic substituent is a group of the formula -NHCH(COOH)(CH₂)₄NH-CO(CH₂)ᵤCH₃, wherein u is an integer of from 8 to 18.

In another preferred embodiment, the lipophilic substituent is a group of the formula -NHCH(COOH)(CH₂)₄NH-COCH((CH₂)₂COOH)NH-CO(CH₂)_{w}CH₃, wherein w is an integer of from 10 to 16.

In another preferred embodiment, the lipophilic substituent is a group of the formula -NHCH(COOH)(CH₂)₄NH-CO(CH₂)₂CH(COOH)NH-CO(CH₂)ₓCH₃, wherein x is an integer of from 10 to 16.

In another preferred embodiment, the lipophilic substituent is a group of the formula -NHCH(COOH)(CH₂)₄NH-CO(CH₂)₂CH(COOH)NH-CO(CH₂)_{y}CH₃, wherein y is zero or an integer of from 1 to 22.

In a preferred embodiment the pharmaceutical composition according to the invention, comprises a GLP-1 derivative wherein the parent peptide is GLP-1(A-B) wherein A is an integer from 1 to 7 and B is an integer from 38 to 45, or an analogue thereof.

The parent peptide is preferably, in one embodiment, selected from the group comprising GLP-1(7-35); GLP-1(7-36); GLP-1(7-36)amide; GLP-1(7-37); GLP-1(7-38); GLP-1(7-39); GLP-1(7-40) and GLP-1(7-41); and analogues thereof.

The parent peptide is preferably, in another embodiment, selected from the group comprising GLP-1(1-35); GLP-1(1-36); GLP-1(1-36)amide; GLP-1(1-37); GLP-1(1-38); GLP-1(1-39); GLP-1(1-40); GLP-1(1-41); and an analogues thereof.

In yet another embodiment, the parent peptide is a GLP-1 analogue of formula I: 
7 8 9 10 11 12 13 14 15 16 17
His-Xaa-Xaa-Gly-Xaa-Phe-Thr-Xaa-Asp-Xaa-Xaa-

18 19 20 21 22 23 24 25 26 27 28
Xaa-Xaa-Xaa-Xaa-Xaa-Xaa-Xaa-Xaa-Xaa-Xaa-Phe-

29 30 31 32 33 34 35 36 37 38
Ile-Xaa-Xaa-Xaa-Xaa-Xaa-Xaa-Xaa-Xaa-Xaa

39 40 41 42 43 44 45
Xaa-Xaa-Xaa-Xaa-Xaa-Xaa-Xaa

(I)
 wherein
Xaa at position 8 is Ala, Gly, Ser, Thr, Leu, Ile, Val, Glu, Asp, or Lys,
Xaa at position 9 is Glu, Asp, or Lys,
Xaa at position 11 is Thr, Ala, Gly, Ser, Leu, Ile, Val, Glu, Asp, or Lys,
Xaa at position 14 is Ser, Ala, Gly, Thr, Leu, Ile, Val, Glu, Asp, or Lys,
Xaa at position 16 is Val, Ala, Gly, Ser, Thr, Leu, Ile, Tyr, Glu, Asp, or Lys,
Xaa at position 17 is Ser, Ala, Gly, Thr, Leu, Ile, Val, Glu, Asp, or Lys,
Xaa at position 18 is Ser, Ala, Gly, Thr, Leu, Ile, Val, Glu, Asp, or Lys,
Xaa at position 19 is Tyr, Phe, Trp, Glu, Asp, or Lys,
Xaa at position 20 is Leu, Ala, Gly, Ser, Thr, Leu, Ile, Val, Glu, Asp, or Lys,
Xaa at position 21 is Glu, Asp, or Lys,
Xaa at position 22 is Gly, Ala, Ser, Thr, Leu, Ile, Val, Glu, Asp, or Lys,
Xaa at position 23 is Gln, Asn, Arg, Glu, Asp, or Lys,
Xaa at position 24 is Ala, Gly, Ser, Thr, Leu, Ile, Val, Arg, Glu, Asp, or Lys,
Xaa at position 25 is Ala, Gly, Ser, Thr, Leu, Ile, Val, Glu, Asp, or Lys,
Xaa at position 26 is Lys, Arg, Gln, Glu, Asp, or His,
Xaa at position 27 is Glu, Asp, or Lys,
Xaa at position 30 is Ala, Gly, Ser, Thr, Leu, Ile, Val, Glu, Asp, or Lys,
Xaa at position 31 is Trp, Phe, Tyr, Glu, Asp, or Lys,
Xaa at position 32 is Leu, Gly, Ala, Ser, Thr, Ile, Val, Glu, Asp, or Lys,
Xaa at position 33 is Val, Gly, Ala, Ser, Thr, Met, Leu, Ile, Glu, Asp, or Lys,
Xaa at position 34 is Lys, Arg, Glu, Asp, or His,
Xaa at position 35 is Gly, Ala, Ser, Thr, Leu, Ile, Val, Glu, Asp, or Lys,
Xaa at position 36 is Arg, Lys, Glu, Asp, or His, or
Xaa at position 37 is Gly, Ala, Ser, Thr, Leu, Ile, Val, Glu, Asp, or Lys, or is deleted,
Xaa at position 38 is Arg, Lys, Glu, Asp, or His, or is deleted,
Xaa at position 39 is Arg, Lys, Glu, Asp, or His, or is deleted,
Xaa at position 40 is Asp, Glu, or Lys, or is deleted,
Xaa at position 41 is Phe, Trp, Tyr, Glu, Asp, or Lys, or is deleted,
Xaa at position 42 is Pro, Lys, Glu, or Asp, or is deleted,
Xaa at position 43 is Glu, Asp, or Lys, or is deleted,
Xaa at position 44 is Glu, Asp, or Lys, or is deleted, and
Xaa at position 45 is Val, Glu, Asp, or Lys, or is deleted, or
a C-1-6-ester, amide, C-1-6-alkylamide, C-1-6-dlalkylamide and/or pharmaceutically acceptable salt thereof,
provided that
(i) when the amino acid at position 37, 38, 39, 40, 41, 42, 43 or 44 is deleted, then each amino acid downstream of the amino add is also deleted,
(ii) the derivative of the GLP-1 analog contains only one Lys,
(iii) the ε-amino group of the Lys is substituted with a lipophilic substituent,
(iv) the total number of different amino acids between the derivative of the GLP-1 analog and the corresponding native form of GLP-1 does not exceed six.

The pharmaceutical composition according to the invention preferably comprises a GLP-1 derivative wherein a total of up to fifteen, preferably up to ten, more preferably up to six, amino acid residues have been exchanged with any α-amino acid residue which can be coded for by the genetic code.

The parent peptide is most preferably selected from one of the following the groups
- Arg²⁶-GLP-1(7-37); Arg³⁴-GLP-1(7-37); Lys³⁶-GLP-1(7-37); Arg^{26,34}Lys³⁶-GLP-1(7-37); Arg^{26,34}Lys³⁸GLP-1(7-38); Arg^{26,34}Lys³⁹-GLP-1(7-39); Arg^{26,34}Lys⁴⁰-GLP-1(7-40); Arg²⁶Lys³⁶-GLP-1(7-37); Arg³⁴Lys³⁶-GLP-1(7-37); Arg²⁶Lys³⁹-GLP-1(7-39); Arg³⁴Lys⁴⁰-GLP-1(7-40); Arg^{26,34}Lys^{36,39}-GLP-1(7-39); Arg^{26,34}Lys^{36,40}-GLP-1(7-40); Gly⁸Arg26-GLP-1(7-37); Gly⁸Arg³⁴-GLP-1(7-37); Gly⁸Lys³⁶-GLP-1(7-37); Gly⁸Arg^{26,34}Lys³⁶-GLP-1(7-37); Gly⁸Arg^{26,34}Lys³⁹-GLP-1(7-39); Gly⁸Arg^{26,34}Lys⁴⁰-GLP-1(7-40); Gly⁸Arg²⁶Lys³⁶-GLP-1(7-37); Gly⁸Arg³⁴Lys³⁶-GLP-1(7-37); Gly⁸Arg²⁶Lys³⁹-GLP-1(7-39); Gly⁸Arg³⁴Lys⁴⁰-GLP-1(7-40); Gly⁸Arg^{26,34}Lys^{36,39}-GLP-1(7-39) and Gly⁸Arg^{26,34}Lys^{36,40}-GLP-1(7-40); or
- Arg^{26,34}Lys³⁸GLP-1(7-38); Arg^{26,34}Lys³⁹GLP-1(7-39); Arg^{26,34}Lys⁴⁰GLP-1(7-40); Arg^{26,34}Lys⁴¹GLP-1(7-41); Arg^{26,34}Lys⁴²GLP-1(7-42); Arg^{26,34}Lys⁴³GLP-1(7-43); Arg^{26,34}Lys⁴⁴GLP-1(7-44); Arg^{26,34}Lys⁴⁵GLP-1(7-45); Arg^{26,34}Lys³⁸GLP-1(1-38); Arg^{26,34}Lys³⁹GLP-1(1-39); Arg^{26,34}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1(1-44); Arg^{26,34}Lys⁴⁵GLP-1(1-45); Arg^{26,34}Lys³⁸GLP-1(2-38); Arg^{26,34}Lys³⁹GLP-1(2-39); Arg^{26,34}Lys⁴⁰GLP-1(2-40); Arg^{26,34}Lys⁴¹GLP-1(2-41); Arg^{26,34}Lys⁴²GLP-1(2-42); Arg^{26,34}Lys⁴³GLP-1(2-43); Arg^{26,34}Lys⁴⁴GLP-1(2-44); Arg^{26,34}Lys⁴⁵GLP-1(2-45); Arg^{26,34}Lys³⁸GLP-1(3-38); Arg^{26,34}Lys³⁹GLP-1(3-39); Arg^{26,34}Lys⁴⁰GLP-1(3-40); Arg^{26,34}Lys⁴¹GLP-1(3-41); Arg^{26,34}Lys⁴²GLP-1(3-42); Arg^{26,34}Lys⁴³GLP-1(3-43); Arg^{26,34}Lys⁴⁴GLP-1(3-44); Arg^{26,34}Lys⁴⁵GLP-1(3-45); Arg^{26,34}Lys³⁸GLP-1(4-38); Arg^{26,34}Lys³⁹GLP-1(4-39); Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,34}Lys⁴⁵GLP1(4-45); Arg^{26,34}Lys³⁸GLP-1(5-38); Arg^{26,34}Lys³⁹GLP-1(5-39); Arg^{26,34}Lys⁴⁰GLP-1(5-40); Arg^{26,34}Lys⁴¹GLP-1(5-41); Arg^{26,34}Lys⁴²GLP-1(5-42); Arg^{26,34}Lys⁴³GLP-1(5-43); Arg^{26,34}Lys⁴⁴GLP-1(5-44); Arg^{26,34}Lys⁴⁵GLP-1(5-45); Arg^{26,34}Lys³⁸GLP-1(6-38); Arg^{26,34}Lys³⁹GLP-1(6-39); Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁸GLP-1(1-38); Arg³⁴Lys³⁸GLP-1(1-38); Arg^{26,34}Lys^{36,38}GLP-1(1-38); Arg²⁶Lys³⁸GLP-1(7-38); Arg³⁴Lys³⁸GLP-1(7-38); Arg^{26,34}Lys^{36,38}GLP-1(7-38); Arg^{26,34}Lys³⁸GLP-1(7-38); Arg²⁶Lys³⁹GLP-1(1-39); Arg³⁴Lys³⁹GLP-1(1-39); Arg^{26,34}Lys^{36,39}GLP-1(1-39); Arg²⁶Lys³⁹GLP-1(7-39); Arg³⁴Lys³⁹GLP-1(7-39) and Arg^{26,34}Lys^{36,39}GLP-1(7-39).

The present invention furthermore relates to a method for improving the solubility and/or stability of GLP-1 or a fragment or an analogue thereof, characterised in that a lipophilic substituent is introduced on any one of the amino acid residues of the parent peptide.

By this method the lipophilic substituent is preferably introduced on any one of the amino acid residues in position 18-38, preferably 26-34.

The lipophilic substituent preferably comprises from 4 to 40 carbon atoms, more preferably from 8 to 25 carbon atoms.

In a preferred embodiment the lipophilic substituent is the acyl group of a straight-chain or branched fatty acid; preferably selected from the group comprising CH₃(CH₂)ₙCO-, wherein n is 4 to 38, preferably CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

The GLP-1 parent peptide is preferably GLP-1(A-B) wherein A is an integer from 1 to 7 and B is an integer from 38 to 45, or an analogue thereof.

In one preferred embodiment, the GLP-1 is selected from the group comprising GLP-1(7-35); GLP-1(7-36); GLP-1(7-36)amide; GLP-1(7-37); GLP-1(7-38); GLP-1(7-39); GLP-1(7-40) and GLP-1(7-41); and analogues thereof.

In another preferred embodiment, the GLP-1 is selected from the group comprising GLP-1(1-35); GLP-1(1-36); GLP-1(1-36)amide; GLP-1(1-37); GLP-1(1-38); GLP-1(1-39); GLP-1(1-40); GLP-1(1-41); and an analogues thereof.

### Brief description of the drawing

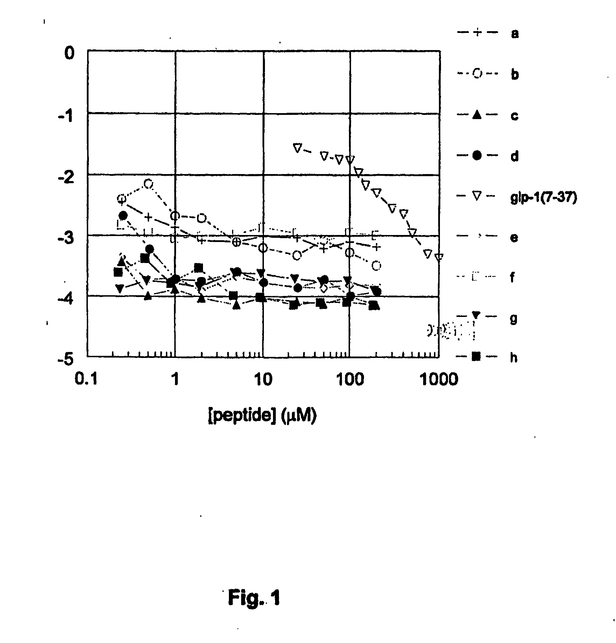

Figure 1: Circular Dichroism (CD) at 222 nm as a function of peptide concentration for acylated GLP-1 derivatives dissolved in 10 mM tris buffer, pH 8, and 23°C.

### Detailed description of the invention

To obtain a satisfactory protracted profile of action of the GLP-1 derivative, the lipophilic substituent attached to the GLP-1 moiety preferably comprises 4-40 carbon atoms, in particular 8-25 carbon atoms. The lipophilic substituent may be attached to an amino group of the GLP-1 moiety by means of a carboxyl group of the lipophilic substituent which forms an amide bond with an amino group of the amino acid residue to which it is attached. Alternatively, the lipophilic substituent may be attached to said amino acid residue in such a way that an amino group of the lipophilic substituent forms an amide bond with a carboxyl group of the amino acid residue. As a further option, the lipophilic substituent may be linked to the GLP-1 moiety via an ester bond. Formally, the ester can be formed either by reaction between a carboxyl group of the GLP-1 moiety and a hydroxyl group of the substituent-to-be or by reaction between a hydroxyl group of the GLP-1 moiety and a carboxyl group of the substituent-to-be. As a further alternative, the lipophilic substituent can be an alkyl group which is introduced into a primary amino group of the GLP-1 moiety.

In one preferred embodiment of the invention, the lipophilic substituent is attached to the GLP-1 moiety by means of a spacer in such a way that a carboxyl group of the spacer forms an amide bond with an amino group of the GLP-1 moiety. Examples of suitable spacers are succinic acid, Lys, Glu or Asp, or a dipeptide such as Gly-Lys. When the spacer is succinic acid, one carboxyl group thereof may form an amide bond with an amino group of the amino acid residue, and the other carboxyl group thereof may form an amide bond with an amino group of the lipophilic substituent. When the spacer is Lys, Glu or Asp, the carboxyl group thereof may form an amide bond with an amino group of the amino acid residue, and the amino group thereof may form an amide bond with a carboxyl group of the lipophilic substituent. When Lys is used as the spacer, a further spacer may in some instances be inserted between the ε-amino group of Lys and the lipophilic substituent. In one preferred embodiment, such a further spacer is succinic acid which forms an amide bond with the ε-amino group of Lys and with an amino group present in the lipophilic substituent. In another preferred embodiment such a further spacer is Glu or Asp which forms an amide bond with the ε-amino group of Lys and another amide bond with a carboxyl group present in the lipophilic substituent, that is, the lipophilic substituent is a N^{ε}-acylated lysine residue.

In another preferred embodiment of the present invention, the lipophilic substituent has a group which can be negatively charged. One preferred group which can be negatively charged is a carboxylic acid group.

The parent peptide can be produced by a method which comprises culturing a host cell containing a DNA sequence encoding the polypeptide and capable of expressing the polypeptide in a suitable nutrient medium under conditions permitting the expression of the peptide, after which the resulting peptide is recovered from the culture.

The medium used to culture the cells may be any conventional medium suitable for growing the host cells, such as minimal or complex media containing appropriate supplements. Suitable media are available from commercial suppliers or may be prepared according to published recipes (*e*.*g*. in catalogues of the American Type Culture Collection). The peptide produced by the cellls may then be recovered from the culture medium by conventional procedures including separating the host cells from the medium by centrifugation or filtration, precipitating the proteinaceous components of the supernatant or filtrate by means of a salt, *e.g.* ammonium sulphate, purification by a variety of chromatographic procedures, *e*.*g*. ion exchange chromatography, gel filtration chromatography, affinity chromatography, or the like, dependent on the type of peptide in question.

The DNA sequence encoding the parent peptide may suitably be of genomic or cDNA origin, for instance obtained by preparing a genomic or cDNA library and screening for DNA sequences coding for all or part of the peptide by hybridisation using synthetic oligonucleotide probes in accordance with standard techniques (see, for example, Sambrook, J, Fritsch, EF and Maniatis, T, *Molecular Cloning: A Laboratory Manual*, Cold Spring Harbor Laboratory Press, New York, 1989). The DNA sequence encoding the peptide may also be prepared synthetically by established standard methods, *e*.*g*. the phosphoamidite method described by Beaucage and Caruthers, *Tetrahedron Letters* **22** (1981), 1859 - 1869, or the method described by Matthes *et al*., *EMBO Journet* **3** (1984), 801 - 805. The DNA sequence may also be prepared by polymerase chain reaction using specific primers, for instance as described in US 4,683,202 or Saiki *et al*., *Science* **239** (1988), 487 - 491.

The DNA sequence may be inserted into any vector which may conveniently be subjected to recombinant DNA procedures, and the choice of vector will often depend on the host cell into which it is to be introduced. Thus, the vector may be an autonomously replicating vector, *i*.*e*. a vector which exists as an extrachromosomal entity, the replication of which is independent of chromosomal replication, *e*.*g*. a plasmid. Altematively, the vector may be one which, when introduced into a host cell, is integrated into the host cell genome and replicated together with the chromosome(s) into which it has been integrated.

The vector is preferably an expression vector in which the DNA sequence encoding the peptide is operably linked to additional segments required for transcription of the DNA, such as a promoter. The promoter may be any DNA sequence which shows transcriptional activity in the host cell of choice and may be derived from genes encoding proteins either homologous or heterologous to the host cell. Examples of suitable promoters for directing the transcription of the DNA encoding the peptide of the invention in a variety of host cells are well known in the art, cf. for instance Sambrook *et al*., *supra.*

The DNA sequence encoding the peptide may also, if necessary, be operably connected to a suitable terminator, polyadenylation signals, transcriptional enhancer sequences, and translational enhancer sequences. The recombinant vector of the invention may further comprise a DNA sequence enabling the vector to replicate in the host cell in question.

The vector may also comprise a selectable marker, *e.g.* a gene the product of which complements a defect in the host cell or one which confers resistance to a drug, *e.g.* ampicillin, kanamycin, tetracyclin, chloramphenicol, neomycin, hygromycin or methotrexate.

To direct a parent peptide of the present invention into the secretory pathway of the host cells, a secretory signal sequence (also known as a leader sequence, prepro sequence or pre sequence) may be provided in the recombinant vector. The secretory signal sequence is joined to the DNA sequence encoding the peptide in the correct reading frame. Secretory signal sequences are commonly positioned 5' to the DNA sequence encoding the peptide. The secretory signal sequence may be that normally associated with the peptide or may be from a gene encoding another secreted protein.

The procedures used to ligate the DNA sequences coding for the present peptide, the promoter and optionally the terminator and/or secretory signal sequence, respectively, and to insert them into suitable vectors containing the information necessary for replication, are well known to persons skilled in the art (cf., for instance, Sambrook *et al*.., *supra*).

The host cell into which the DNA sequence or the recombinant vector is introduced may be any cell which is capable of producing the present peptide and includes bacteria, yeast, fungi and higher eukaryotic cells. Examples of suitable host cells well known and used in the art are, without limitation, *E. coli, Saccharomyces cerevisiae*, or mammalian BHK or CHO cell lines.

Examples of compounds which can be useful as GLP-1 moieties according to the present invention are described in International Patent Application No. WO 87/06941 (The General Hospital Corporation) which relates to a peptide fragment which comprises GLP-1(7-37) and functional derivatives thereof and to its use as an insulinotropic agent.

Further GLP-1 analogues are described in international Patent Application No. 90/11296 (The General Hospital Corporation) which rolates to peptide fragments which comprise GLP-1(7-36) and functional derivatives thereof and have an insulinotropic activity which exceeds the insulinotropic activity of GLP-1(1-36) or GLP-1(1-37) and to their use as insulinotropic agents.

International Patent Application No. 91/11457 (Buckley *et al*..) discloses analogues of the active GLP-1 peptides 7-34, 7-35, 7-36, and 7-37 which can also be useful as GLP-1 moieties according to the present invention.

### Preparation and administration of the compositions

Pharmaceutical compositions containing a GLP-1 derivative according to the present invention may be administered parenterally or orally to patients in need of such a treatment. Parenteral administration may be performed by subcutaneous, intramuscular or intravenous infection by means of a syringe, optionally a pen-like syringe. Alternatively, parenteral administration can be performed by means of an infusion pump. A further option is a composition which may be a powder or a liquid for the administration of the GLP-1 derivative in the form of a nasal or pulmonal spray. As a still further option, the pharmaceutical compositions containing a the GLP-1 derivatives of the invention can also be adapted to transdermal administration, *e.g*. from a patch, optionally a iontophoretic patch, or transmucosal, *e*.*g*. bucal, administration.

Pharmaceutical compositions containing a GLP-1 derivative of the present invention may be prepared by conventional techniques, *e*.*g*. as described in Remington's *Pharmaceutical Sciences*, 1985 or in Remington: *The Science and Practice of Pharmacy,* 19^{th} edition, 1995.

Thus, the injectable compositions of the GLP-1 derivative of the invention can be prepared using the conventional techniques of the pharmaceutical industry which involves dissolving and mixing the ingredients as appropriate to give the desired end product.

According to one procedure, the GLP-1 derivative is dissolved in an amount of water which is somewhat less than the final volume of the composition to be prepared. An isotonic agent, a preservative and a buffer is added as required and the pH value of the solution is adjusted - if necessary - using an acid, *e*.*g*. hydrochloric acid, or a base, *e.g.* aqueous sodium hydroxide as needed. Finally, the volume of the solution is adjusted with water to give the desired concentration of the ingredients.

A composition for nasal administration of certain peptides may, for example, be prepared as described in European Patent No. 272097 (to Novo Nordisk A/S) or in WO 93/18785.

According to one preferred embodiment of the present invention, the pharmaceutical compositions containing a GLP-1 derivative is provided in the form of a composition suitable for administration by injection. Such a composition can either be an injectable solution ready for use or it can be an amount of a solid composition, *e.g.* a lyophilised product, which has to be dissolved in a solvent before it can be injected. The injectable solution preferably contains not less than about 0.5 mg/ml, preferably not less than about 5 mg/ml, more preferred not less than about 10 mg/ml of the GLP-1 derivative and, preferably, not more than about 100 mg/ml of the GLP-1 derivative.

The pharmaceutical compositions containing a GLP-1 derivative of this invention can be used in the treatment of various diseases. The particular GLP-1 derivative to be used and the optimal dose level for any patient will depend on the disease to be treated and on a variety of factors including the efficacy of the specific peptide derivative employed, the age, body weight, physical activity, and diet of the patient, on a possible combination with other drugs, and on the severity of the case. It is recommended that the dosage of the GLP-1 derivative of this invention be determined for each individual patient by those skilled in the art.

In particular, it is envisaged that the pharmaceutical compositions containing a GLP-1 derivative will be useful for the treatment of non-insulin dependent diabetes mellitus and/or for the treatment of obesity.

The present invention is further illustrated by the following examples which, however, are not to be construed as limiting the scope of protection.

### Examples

Circular Dichroism (CD) at 222 nm as a function of peptide concentration for peptides dissolved in 10 mM tris buffer, pH 8, and 23°C was measured for GLP-1 (7-37) and the following eight GLP-1 derivatives suitable for pharmaceutical formulations according to the present invention:

| *Derivative* | *Position of lipophilic substituent* |
|---|---|
| (a) | 26 |
| (b) | 26 |
| (c) | 23 |
| (d) | 34 |
| (e) | 38 |
| (f) | 26 |
| (g) | 26 |
| (h) | 18 |

| | |
|---|---|
| (a) is Arg³⁴Lys²⁶(N^{ε}-(γ-glutamyl(N^{α}-hexadecanoyl)))-GLP-1(7-37)-OH; | |
| (b) is Arg³⁴,Lys²⁶ (N^{ε}-(γ-glutamyl(N^{α}-lithochoyl))) GLP-1(7-37)-OH; | |
| (c) is Arg^{26,34},Lys²³ (N^{ε}-(γ-glutamyl(N^{α}-hexedecanoyl))) GLP-1 (7-37)-OH; | |
| (d) is Arg²⁶,Lys³⁴ (N^{ε}-(γ-glutamyl(N^{α}-hexadecanoyl))) GLP-1(7-37)-OH; | |
| (e) is Gly⁸,Glu³⁷,Arg^{26,34},Lys³⁸ (N^{ε}-(γ-glutamyl(N^{α}-octadecanoyl))) GLP-1(7-38)-OH; | |
| (f) is Arg³⁴,Lys²⁶ (N^{ε}-(hexadecanoyl)) GLP-1 (7-37)-OH; | |
| (g) is Arg³⁴,Lys²⁶ (N^{ε}-(γ-aminobutyroyl(N^{γ}-hexadecanoyl))) GLP-1 (7-37)-OH; | |
| (h) is Arg^{26,34},Lys¹⁸ (N^{ε}-(γ-glutamyl(N^{α}-hexadecanoyl))) GLP-1 (7-37)-OH. | |

The results are presented in Figure 1. Note that the CD signal is proportional to the average content of α-helix in the peptides, i.e., a CD value of -1 corresponds to 10% α-helix content under these conditions. The figure shows that, as the concentration of unmodified GLP-1(7-37) is raised between 25 and 1000 µM, the content of α-helix increases form about 15% to about 30-35% in parallel with the formation of higher oligomers. In contrast with this concentration dependent behaviour, the figure shows that the helix content remains high and essentially independent of the concentration in the 1-200 µM range for a series of acylated glp-1 derivatives forming partially structured micelle-like aggregates under the same conditions.

The following acronyms for commercially available chemicals are used:
- DMF :: N,N-Dimethylformamide.
- DCC :: N,N-Dicyclohexylcarbodiimide
- NMP :: N-Methyl-2-pyrrolidone.
- EDPA :: N-Ethyl-N,N-diisopropylamine.
- EGTA :: Ethylene glycol-bis(β-aminoethyl ether)-N,N,N',N'-tetraacetic acid.
- GTP: Guanosine 5'-triphosphate.
- TFA :: Trifluoroacetic acid.
- THF :: Tetrahydrofuran
- H-Glu(OH)-OBu^{t}:: L-Glutamic acid α-tert-butyl ester
- Cap-ONSu:: Octanoic acid 2,5-dioxopyrrolidin-1-yl ester
- Lau-ONSu:: Dodecanoic acid 2,5-dioxopyrrolidin-1-yl ester
- Myr-ONSu:: Tetradecanoic acid 2,5-dioxopyrrolidin-1-yl ester.
- Pal-ONSu:: Hexadecanoic acid 2,5-dioxopyrrolidin-1-yl ester.
- Ste-ONSu: Octadecanoic acid 2,5-dioxopyrrolidin-1-yl ester.

### Abbreviations:

PDMS: Plasma Desorption Mass Spectrometry
MALDI-MS: Matrix Assisted Laser Desorption/lonisation Mass Spectrometry
HPLC: High Performance Liquid Chromatography
amu: atomic mass units
Lit-Glu(ONSu)-OBu^{t}: N^{α}-Lithochoyl-L-glutamic acid α-t-butyl ester γ-2,5-dioxopyrrolidin-1-yl ester
Cap-Glu(ONSu)-OBu^{t}: N^{α}-Octanoyl-L-glutamic acid α-t-butyl ester γ-2,5-dioxopyrrolidin-1-yl ester
Cac-Glu(ONSu)-OBu^{t}: N^{α}-Decanoyl-L-glutamic acid α-t-butyl ester γ-2,5-dioxopyrrolidin-1-yl ester
Lau-Glu(ONSu)-OBu^{t}: N^{α}-Dodecanoyl-L-glutamic acid α-t-butyl ester γ-2,5-dioxopyrrolidin-1-yl ester
Myr-Glu(ONSu)-OBu^{t}: N^{α}-Tetradecanoyl-L-glutamic acid α-t-butyl ester γ-2,5-dioxopyrrolidin-1-yl ester
Pal-Glu(ONSu)-OBu^{t}: N^{α}-Hexadecanoyl-(L)-glutamic acid α-t-butyl-γ-2,5-dioxopyrrolidin-1-yl diester.
Ste-Glu(ONSu)-OBu^{t}: N^{α}-Octadecanoyl-(L)-glutamic acid α-t-butyl-γ-2,5-dioxopyrrolidin-1-yl diester
Lau-β-Ala-ONSu: N^{β}-Dodecanoyl-β-alanine 2,5-dioxopyrrolidin-1-yl ester
Pal-β-Ala-ONSu: N^{β}-Hexadecanoyl-β-alanine 2,5-dioxopyrrolidin-1-yl ester
Lau-GABA-ONSu: N^{γ}-Dodecanoyl-γ-aminobutyric acid 2,5-dioxopyrrolidin-1-yl ester
Myr-GABA-ONSu: N^{γ}-Tetradecanoyl-γ-aminobutyric acid 2,5-dioxopyrrolidin-1-yl ester
Pal-GABA-ONSu: N^{γ}-Hexadecanoyl-γ-aminobutyric acid 2,5-dioxopyrrolidin-1-yl ester
Ste-GABA-ONSu: N^{γ}-Octadecanoyl-γ-aminobutyric acid 2,5-dioxopyrrolidin-1-yl ester
Pal-Iscinip-ONSu: N-Hexadecanoyl-piperidine-4-carboxylic acid 2,5-dioxopyrrolidin-1-yl ester
Pal-Glu(OBu^{t})-ONSu: N^{α}-Hexadecanoyl-L-glutamic acid α-2,5-dioxopyrrolidin-1-yl ester γ-t-butyl ester
HOOC-(CH₂)₆-COONSu: ω-Carboxyheptanoic acid 2,5-dioxopyrrolidin-1-yl ester.
HOOC-(CH₂)₁₀-COONSu: ω-Carboxyundecanoic acid 2,5-dioxopyrrolidin-1-yl ester.
HOOC-(CH₂)₁₂-COONSu: ω-Carboxytridecanoic acid 2,5-dioxopyrrolidin-1-yl ester.
HOOC-(CH₂)₁₄-COONSu: ω-Carboxypentadecanoic acid 2,5-dioxopyrrolidin-1-yl ester.
HOOC-(CH₂)₁₆-COONSu: ω-Carboxyheptadecanoic acid 2,5-dioxopyrrolidin-1-yl ester.
HOOC-(CH₂)₁₈-COONSu: ω-Carboxynonadecanoic acid 2,5-dioxopyrrolidin-1-yl ester.

### Analytical

### Plasma Desorption Mass Spectrometry

### Sample preparation:

The sample is dissolved in 0.1 % TFA/EtOH (1:1) at a concentration of 1 µg/µl. The sample solution (5-10 µl) is placed on a nitrocellulose target (Bio-ion AB, Uppsala, Sweden) and allowed to adsorb to the target surface for 2 minutes. The target is subsequently rinsed with 2x25 µl 0.1 % TFA and spin-dried. Finally, the nitrocellulose target is placed in a target carrousel and introduced into the mass spectrometer.

### MS analysis:

PDMS analysis was carried out using a Bio-ion 20 time-of flight instrument (Bio-ion Nordic AB, Uppsala, Sweden). An acceleration voltage of 15 kV was applied and molecular ions formed by bombardment of the nitrocellulose surface with 252-Cf fission fragments were accelerated towards a stop detector. The resulting time-of-flight spectrum was calibrated into a true mass spectrum using the H⁺ and NO⁺ ions at m/z 1 and 30, respectively. Mass spectra were generally accumulated for 1.0x10⁶ fission events corresponding to 15-20 minutes. Resulting assigned masses all correspond to isotopically averaged molecular masses. The accuracy of mass assignment is generally better than 0.1 %.

### MALDI-MS

MALDI-TOF MS analysis was carried out using a Voyager RP instrument (PerSeptive Biosystems Inc., Framingham, MA) equipped with delayed extraction and operated in linear mode. Alpha-cyano-4-hydroxy-cinnamic acid was used as matrix, and mass assignments were based on external calibration.

Further examples of compounds which can be useful as GLP-1 derivatives in the pharmaceutical composition according to the present invention are

### Example 1

### Synthesis of Arg^{26,34}, Lys³⁶ (N^{ε}-(γ-glutamyl(N^{α}-hexadecanoyl))) GLP-1 (7-36)-OH.

To a mixture of Arg^{26,34}, Lys³⁶ GLP-1 (7-36)-OH (12.2 mg, 3.67 µmol), EDPA (13.3 mg, 103 µmol), NMP (1.71 ml) and water (855 µl) was added a solution of Pal-Glu(ONSu)-OBu^{t} (5.94 mg, 11 µmol), prepared as described in PCT application no. PCT/DK97/00340, in NMP (148 µl). The reaction mixture was gently shaken for 5 min. at room temperature, and then allowed to stand for an additional 90 min. at room temperature. The reaction was quenched by the addition of a solution of glycine (6 mg, 81 µmol) in water (0.6 ml). A 0.5 % aqueous solution of ammonium-acetate (38 ml) was added, and the resulting mixture eluted onto a Varian 5g C8 Mega Bond Elut®, the immobilised compound washed with 5% aqueous acetonitril (20 ml), and finally liberated from the cartridge by elution with TFA (25 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (3.1 mg, 23 %) was isolated, and the product was analysed by PDMS. The m/z value for the protonated molecular ion was found to be 3695 +- 3. The resulting molecular weight is thus 3694 +- 3 amu (theoretical value 3694 amu).

### Example 2

### Synthesis of Arg^{26,34},Lys³⁶ (N^{ε}-(γ-glutamyl(N^{α}-octadecanoyl))) GLP-1 (7-36)-OH.

To a mixture of Arg^{26,34},Lys³⁶ GLP-1 (7-36)-OH (12.2 mg, 3.7 µmol), EDPA (13.3 mg, 103 µmol), NMP (1.71 ml) and water (855 µl) was added a solution of Ste-Glu(ONSu)-OBu^{t} (6.25 mg, 11 µmol), prepared as described in PCT application no. PCT/DK97/00340, in NMP (1 ml). The reaction mixture was gently shaken for 5 min. at room temperature, and then allowed to stand for an additional 90 min. at room temperature. The reaction was quenched by the addition of a solution of glycine (6 mg, 81 µmol) in water (0.6 ml). A 0.5 % aqueous solution of ammonium acetate (54 ml) was added, and the resulting mixture eluted onto a Varian 5g C8 Mega Bond Elut®, the immobilised compound washed with 5% aqueous acetonitril (20 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (3.7 mg, 27 %) was isolated, and the product was analysed by PDMS. The m/z value for the protonated molecular ion was found to be 3723 +- 3. The resulting molecular weight is thus 3722 +- 3 amu (theoretical value 3722 amu).

### Example 3

### Synthesis of lithocholic acid 2,5-dioxopyrrolidin-1-yl ester.

To a solution of lithocholic acid (5.44 g, 14.3 mmol) in a mixture of anhydrous THF (120 ml) and anhydrous acetonitril (30 ml) was added N-hydroxysuccinimide (1.78 g, 15 mmol). The mixture was cooled to 10°C, a solution of DCC (3.44 g, 16.7 mmol) in anhydrous THF (30 ml) was added drop wise, and the resulting reaction mixture stirred for 16 h at room temperature. The reaction mixture was filtered and partitioned between dichloromethane (450 ml) and 10% aqueous Na₂CO₃(150 ml). The phases were separated, and the organic phase washed with 10% aqueous Na₂CO₃(150 ml), water (2x150 ml), and dried (MgSO₄). The solvent was concentrated *in vacuo*. The residue was crystallised from a mixture of dichloromethane (30 ml) and n-heptane (30 ml). The precipitate was dried in a vacuum drying oven for 36 h to give the title compound (3.46 g, 51 %).

### Example 4

### Synthesis of Lit-Glu(ONSu)-OBu^{t}.

A suspension of H-Glu(OH)-OBu^{t} (1.28 g, 6.33 mmol), DMF (88 ml) and EDPA (0.82 g, 6.33 mmol) and lithocholic acid 2,5-dioxopyrrolidin-1-yl ester, prepared as described in example 3, was stirred for 16 h at room temperature. The reaction mixture was concentrated *in vacuo* and the residue dissolved in ethyl acetate (40 ml). The resulting solution was washed with 5% aqueous citric acid (2x25 ml), brine (10 ml), and filtered). The solvent was concentrated *in vacuo* and the residue dissolved in DMF (12 ml). The resulting solution was added drop wise to a 10% aqueous solution of citric acid whereby the product precipitates. The precipitate was collected and washed with iced water, and dried *in vacuo.* The crude product was recrystallised from a mixture of n-heptane (40 ml) and 2-propanol (17 ml). The precipitate was dried in a vacuum drying oven for 4 h to give the free acid intermediate.
To a solution of the free acid intermediate in DMF (18 ml) was added hydroxysuccinimide (0.45 g, 3.91 mmol), followed by a solution of DCC (0.73 g, 3.56 mmol) in dichloromethane (18 ml). The resulting mixture was stirred at ambient temperature for 18 h, and then filtered. The filtrate was concentrated *in vacuo* to a solid, and the residue was dissolved in dichloromethane (25 ml), and the filtration repeated, the solvent removed *in vacuo* to give a foam. The residue was dissolved in refluxing n-heptane (35 ml), and the product crystallised b addition of 2-propanol. The precipitate was collected, washed with cold n-heptane, dried at 35°C *in vacuo* to give the title compound (1.34 g, 57%).

### Example 5

### Synthesis of Arg³⁴,Lys²⁶ (N^{ε}-(γ-glutamyl(N^{α}-lithochoyl))) GLP-1 (7-37)-OH.

To a mixture of Arg³⁴,Lys²⁶ GLP-1 (7-37)-OH (41.1 mg, 12.2 µmol), EDPA (44 mg, 340 µmol), NMP (5.76 ml) and water (2.88 ml) was added a solution of Lit-Glu(ONSu)-OBu¹ (24 mg, 37 µmol), prepared as described in example 4, in NMP (600 µl). The reaction mixture was gently shaken for 5 min. at room temperature, and then allowed to stand for an additional 75 min. at room temperature. The reaction was quenched by the addition of a solution of glycine (20 mg, 268 µmol) in water (2 ml). A 0.5 % aqueous solution of ammonium acetate (128 ml) was added, and the resulting mixture divided into two equal portions, and each portion eluted onto a Varian 5g C8 Mega Bond Elut®, the immobilised compound washed with 5% aqueous acetonitril (2x25 ml), and finally liberated from the cartridge by elution with TFA (2x25 ml). The combined eluates were concentrated *in vacuo*, and the residue purified by column chromato-graphy using a cyanopropyl column (Zorbax 300SB-CN) and a standard acetonitril/TFA system. The column was heated to 65°C and the acetonitril gradient was 0-100% in 60 minutes. The title compound (5 mg, 11 %) was isolated, and the product was analysed by PDMS. The m/z value for the protonated molecular ion was found to be 3872 +- 3. The resulting molecular weight is thus 3871 +- 3 amu (theoretical value 3871 amu).

### Example 6

### Synthesis of Arg²⁶,Lys³⁴ (N^{ε}-(γ-glutamyl(N^{α}-hexadecanoyl))) GLP-1 (7-37)-OH

To a mixture of Arg²⁶,Lys³⁴ GLP-1 (7-37)-OH (18 mg, 5.3 µmol), EDPA (19.3 mg, 149 µmol), NMP (2.52 ml) and water (1.26 ml) was added a solution of Pal-Glu(ONSu)-OBu^{t} (8.6 mg, 16 µmol) in NMP (215 µl). The reaction mixture was gently shaken for 5 min. at room temperature, and then allowed to stand for an additional 90 min. at room temperature. The reaction was quenched by the addition of a solution of glycine (8.8 mg, 117 µmol) in water (0.88 ml). A 0.5 % aqueous solution of ammonium acetate (50 ml) was added, and the resulting mixture eluted onto a Varian 5g C8 Mega Bond Elut®, the immobilised compound washed with 5% aqueous acetonitril (25 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (6 mg, 30 %) was isolated, and the product was analysed by PDMS. The m/z value for the protonated molecular ion was found to be 3752 +-3. The resulting molecular weight is thus 3751 +- 3 amu (theoretical value 3751 amu).

### Example 7

### Synthesis of desamino-His⁷,Arg²⁶,Lys³⁴(N^{ε}-(γ-glutamyl(N^{α}-hexadecanoyl))) GLP-1 (7-37)-OH.

To a mixture of desamino-His⁷,Arg²⁶,Lys³⁴ GLP-1 (7-37)-OH (14.3 mg, 4.2 µmol), EDPA (15.3 mg, 119 µmol), NMP (2 ml) and water (1 ml) was added a solution of Pal-Glu(ONSu)-OBu^{t} (6.84 mg, 12.7 µmol) in NMP (171 µl). The reaction mixture was gently shaken for 5 min. at room temperature, and then allowed to stand for an additional 50 min. at room temperature. The reaction was quenched by the addition of a solution of glycine (7 mg, 99 µmol) in water (700 µl). A 0.5 % aqueous solution of ammonium acetate (42 ml) was added, and the resulting mixture eluted onto a Varian 5g C8 Mega Bond Elut®, the immobilised compound washed with 5% aqueous acetonitril (25 ml), and finally liberated from the cartridge by elution with TFA (25 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (5.6 mg, 35 %) was isolated, and the product was analysed by PDMS. The m/z value for the protonated molecular ion was found to be 3738 +- 3. The resulting molecular weight is thus 3737 +- 3 amu (theoretical value 3737 amu).

### Example 8

### Synthesis of Gly⁸,Arg^{26,34},Lys³⁸ (N^{ε}-(γ-glutamyl(N^{α}-hexadecanoyl))) GLP-1 (7-38)-OH.

To a mixture of Gly⁸,Arg^{26,34},Lys³⁸ GLP-1 (7-38)-OH (11.8 mg, 3.4 µmol), EDPA (12.1 mg, 94 µmol), NMP (1.65 ml) and water (0.83 ml) was added a solution of Pal-Glu(ONSu)-OBu^{t} (5.4 mg, 10 µmol) in NMP (135 µl). The reaction mixture was gently shaken for 5 min. at room temperature, and then allowed to stand for an additional 75 min. at room temperature. The reaction was quenched by the addition of a solution of glycine (5.5 mg, 73.7 µmol) in water (553 µl). A 0.5 % aqueous solution of ammonium acetate (36 ml) was added, and the resulting mixture eluted onto a Varian 5g C8 Mega Bond Elut®, the immobilised compound washed with 5% aqueous acetonitril (25 ml), and finally liberated from the cartridge by elution with TFA (25 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (5 mg, 38 %) was isolated, and the product was analysed by POMS. The m/z value for the protonated molecular ion was found to be 3895 +- 3. The resulting molecular weight is thus 3894 +- 3 amu (theoretical value 3894 amu).

### Example 9

### Synthesis of Gly⁸,Glu³⁷,Arg^{26,34},Lys³⁸ (N^{ε}-(γ-glutamyl(N^{α}-hexadecanoyl))) GLP-1 (7-38)-OH.

To a mixture of Gly⁸,Glu³⁷,Arg^{26,34},Lys³⁸ GLP-1 (7-38)-OH (9 mg, 2.48 µmol), EDPA (9 mg, 69.4 µmol), NMP (1.25 ml) and water (0.63 ml) was added a solution of Pal-Glu(ONSu)-OBu^{t} (4 mg, 7.4 µmol) in NMP (100 µl). The reaction mixture was gently shaken for 5 min. at room temperature, and then allowed to stand for an additional 105 min. at room temperature. The reaction was quenched by the addition of a solution of glycine (4.1 mg, 54.6 µmol) in water (410 µl). A 0.5 % aqueous solution of ammonium acetate (27 ml) was added, and the resulting mixture eluted onto a Varian 5g C8 Mega Bond Elut®, the immobilised compound washed with 5% aqueous acetonitril (15 ml), and finally liberated from the cartridge by elution with TFA (15 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (2.9 mg, 29 %) was isolated, and the product was analysed by PDMS. The m/z value for the protonated molecular ion was found to be 3967 +- 3. The resulting molecular weight is thus 3966 +- 3 amu (theoretical value 3967 amu).

### Example 10

### Synthesis of Gly⁸,Glu³⁷,Arg^{26,34},Lys³⁸ (N^{ε}-(γ-glutamyl(N^{α}-octadecanoyl))) GLP-1 (7-38)-OH.

To a mixture of Gly⁸,Glu³⁷,Arg^{26,34},Lys³⁸ GLP-1 (7-38)-OH (9 mg, 2.5 µmol), EDPA (9 mg, 69.4 µmol), NMP (1.25 ml) and water (0.63 ml) was added a solution of Ste-Glu(ONSu)-OBu^{t} (4.2 mg, 7.4 µmol in NMP (105 µl). The reaction mixture was gently shaken for 5 min. at room temperature, and then allowed to stand for an additional 105 min. at room temperature. The reaction was quenched by the addition of a solution of glycine (4.1 mg, 54.6 µmol) in water (409 µl). A 0.5 % aqueous solution of ammonium acetate (27 ml) was added, and the resulting mixture eluted onto a Varian 5g C8 Mega Bond Elut®, the immobilised compound washed with 5% aqueous acetonitril (15 ml), and finally liberated from the cartridge by elution with TFA (15 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (3.2 mg, 32 %) was isolated, and the product was analysed by PDMS. The m/z value for the protonated molecular ion was found to be 3995 +- 3. The resulting molecular weight is thus 3994 +- 3 amu (theoretical value 3995 amu).

### Example 11

### Synthesis of Cap-Glu(ONSu)-OBu^{t}.

To a solution of octanoic acid (5 g, 34.7 mmol) and N-hydroxysuccinmide (4 g, 34.7 mmol) in anhydrous acetonitril (10 ml) was added a solution of DCC (7.15 g, 34.7 mmol) in anhydrous dichloromethane (15 ml), and the resulting reaction mixture stirred for 16 h at room temperature. The precipitated solid was filtered off and recrystallised from a mixture of n-heptane (40 ml) and 2-propanol (2 ml). The precipitate was dried in a vacuum drying oven for 16 h to give the intermediate Cap-ONSu. A suspension of the crude ester intermediate (3.9 g, 16.2 mmol), (L)-H-Glu(OH)-OBu^{t} (3.28 g, 16.2 mmol), DMF (268 ml) and EDPA (2.1 g, 16.2 mmol) was stirred for 64 h at room temperature. The reaction mixture was concentrated *in vacuo* and the residue dissolved in ethyl acetate (50 ml). The resulting solution was washed with 5% aqueous citric acid (2x25 ml). The solvent was concentrated *in vacuo* and the residue dissolved in DMF (36 ml). The resulting solution was added drop wise to a 10% aqueous solution of citric acid (357 ml) and extracted with ethyl acetate (200 ml), and dried (MgSO₄). The solvent was concentrated *in vacuo* to give the crude glutamic acid intermediate. To a mixture of the crude glutamic acid intermediate, N-hydroxysuccinimide (1.85 g, 16.1 mmol) and DMF (25 ml) was added a solution of DCC (3.32 g, 16.1 mmol) in dichloromethane (15 ml). The resulting mixture was stirred at ambient temperature for 20 h. The reaction mixture was filtered and the solvent concentrated *in vacuo*. The residue was purified on a silica gel column (40- 63µ), eluted with a mixture of dichloromethane and acetonitril (1:1) to give the title compound (0.63 g, 6% over all).

### Example 12

### Synthesis of desamino-His⁷,Arg²⁶,Lys³⁴ (N^{ε}-(γ-glutamyl(N^{α}-octanoyl))) GLP-1 (7-37)-OH.

To a mixture of desamino-His⁷,Arg²⁶,Lys³⁴GLP-1 (7-37)-OH (14.3 mg, 4.2 µmol), EDPA (15.3 mg, 119 µmol), NMP (2 ml) and water (1 ml) was added a solution of Cap-Glu(ONSu)-OBu^{t}. (6.8 mg, 12.7 µmol), prepared as described in example 11, in NMP (135 µl). The reaction mixture was gently shaken for 5 min. at room temperature, and then allowed to stand for an additional 2 h at room temperature. The reaction was quenched by the addition of a solution of glycine (7 mg, 93 µmol) in water (698 µl). A 0.5 % aqueous solution of ammonium acetate (42 ml) was added, and the resulting mixture eluted onto a Varian 5g C8 Mega Bond Elut®, the immobilised compound washed with 5% aqueous acetonitril (25 ml), and finally liberated from the cartridge by elution with TFA (25 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (4.1 mg, 27 %) was isolated, and the product was analysed by PDMS. The m/z value for the protonated molecular ion was found to be 3626 +- 3. The resulting molecular weight is thus 3625 +- 3 amu (theoretical value 3625 amu).

### Example 13

### Synthesis of Glu³⁷,Arg^{26,34},Lys³⁸ (N^{ε}-(γ-glutamyl(N^{α}-hexadecanoyl))) GLP-1 (7-38)-OH.

To a mixture of Glu³⁷,Arg^{26,34},Lys³⁸ GLP-1(7-38)-OH (17.6 mg, 4.9 µmol), EDPA (17.6 mg, 136 µmol), NMP (1.23 ml) and water (2.46 ml) was added a solution of Pal-Glu(ONSu)-OBu^{t} (7.9 mg, 14.6 µmol) in NMP (197 µl). The reaction mixture was gently shaken for 5 min. at room temperature, and then allowed to stand for an additional 2 h at room temperature. The reaction was quenched by the addition of a solution of glycine (8 mg, 107 µmol) in water (804 µl). A 0.5 % aqueous solution of ammonium acetate (49 ml) was added, and the resulting mixture eluted onto a Varian 5g C8 Mega Bond Elut®, the immobilised compound washed with 5% aqueous acetonitril (25 ml), and finally liberated from the cartridge by elution with TFA (25 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (5.1 mg, 26 %) was isolated, and the product was analysed by PDMS. The m/z value for the protonated molecular ion was found to be 3981 +- 3. The resulting molecular weight is thus 3980 +- 3 amu (theoretical value 3981 amu).

### Example 14

### Synthesis of Arg³⁴,Lys²⁶ (N^{ε}-(γ-glutamyl(N^{α}-octadecanoyl))) GLP-1 (7-37)-OH.

To a mixture of Arg³⁴ GLP-1 (7-37)-OH (41.1 mg, 12.2 µmol), EDPA (44 mg, 341 µmol), NMP (5.76 ml) and water (2.88 ml) was added a solution of Ste-Glu(ONSu)-OBu^{t} (20.7 mg, 36.5 µmol in NMP (517 µl). The reaction mixture was gently shaken for 5 min. at room temperature, and then allowed to stand for an additional 2 h at room temperature. The reaction was quenched by the addition of a solution of glycine (20.1 mg, 268 µmol) in water (2.01 ml). A 0.5 % aqueous solution of ammonium-acetate (120 ml) was added, and the resulting mixture eluted onto a Varian 5g C8 Mega Bond Elut®, the immobilised compound washed with 5% aqueous acetonitril (25 ml), and finally liberated from the cartridge by elution with TFA (25 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (15.4 mg, 34 %) was isolated, and the product was analysed by PDMS. The m/z value for the protonated molecular ion was found to be 3781 +- 3. The resulting molecular weight is thus 3780 +- 3 amu (theoretical value 3779 amu).

### Example 15

### Synthesis of Arg³⁴,Lys²⁶(N^{ε}-decanoyl) GLP-1 (7-37).

To a mixture of Arg³⁴-GLP-1 (7-37)-OH (20 mg, 5.9 µmol), EDPA (21.4 mg, 165 µmol), NMP (2.8 ml) and water (1.4 ml) was added a solution of Cac-ONSu (4.8 mg, 17.7 µmol) in NMP (119 µl). The reaction mixture was gently shaken for 5 min., and then allowed to stand for an additional 2h at room temperature. The reaction was quenched by the addition of a solution of glycine (9.8 mg, 130 µmol) in water (98 µl). The resulting mixture was purified by column chromatography using a cyanopropyl column (Zorbax 300SB-CN) and a standard acetonitril/TFA system. The column was heated to 65°C and the acetonitril gradient was 0-100% in 60 minutes. The title compound (7.4 mg, 35%) was isolated, and the product was analysed by PDMS. The m/z value for the protonated molecular ion was found to be 3539.6 ± 3. The resulting molecular weight is thus 3538.6 ± 3 amu (theoretical value 3538 amu).

### Example 16

### Synthesis of Arg³⁴,Lys²⁶ (N^{ε}-(hexadecanoyl)) GLP-1 (7-37)-OH.

To a mixture of Arg³⁴ GLP-1 (7-37)-OH (41.1 mg, 12.2 µmol), EDPA (44 mg, 340 µmol), NMP (2.88 ml) and water (2.88 ml) was added a solution of Pal-ONSu (12.9 mg, 36.5 µmol) in NMP (3.3 ml). The reaction mixture was gently shaken for 5 min. at room temperature, and then allowed to stand for an additional 110 min. at room temperature. The reaction was quenched by the addition of a solution of glycine (20.1 mg, 268 µmol) in water (201 µl). The solvent was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (15 mg, 34 %) was isolated, and the product was analysed by PDMS.

### Example 17

### Synthesis of Arg^{26,34},Lys²⁷ (N^{ε}-(γ-glutamyl(N^{α}-hexadecanoyl))) GLP-1 (7-37)-OH

To a mixture of Arg^{26,34}, Lys²⁷GLP-1 (7-37)-OH (11.6 mg, 3.4 µmol), EDPA (12.3 mg, 94.9 µmol), NMP (1.6 ml) and water (0.8 ml) was added a solution of Pal-Glu(ONSu)-OBu^{t} (5.5 mg, 10.2 µmol) in NMP (137 µl). The reaction mixture was gently shaken for 5 min. at room temperature, and then allowed to stand for an additional 90 min. at room temperature. The reaction was quenched by the addition of a solution of glycine (5.6 mg, 74.6 µmol) in water (560 µl). A 0.5 % aqueous solution of ammonium acetate (34 ml) was added, and the resulting mixture eluted onto a Varian 5g C8 Mega Bond Elut®, the immobilised compound washed with 5% aqueous acetonitril (15 ml), and finally liberated from the cartridge by elution with TFA (25 ml). The solvent was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (2.1 mg, 16 %) was isolated, and the product was analysed by PDMS.

### Example 18

### Synthesis of Arg^{26,34},Lys²³ (N^{ε}-(γ-glutamyl(N^{α}-hexadecanoyl)))GLP-1 (7-37)-OH.

To a mixture of Arg^{26,34}, Lys²³GLP-1 (7-37)-OH (11.6 mg, 3.4 µmol), EDPA (12.3 mg, 94.9 µmol), NMP (1.6 ml) and water (0.8 ml) was added a solution of Pal-Glu(ONSu)-OBu^{t} (5.5 mg, 10.2 µmol) in NMP (137 µl). The reaction mixture was gently shaken for 5 min. at room temperature, and then allowed to stand for an additional 90 min. at room temperature. The reaction was quenched by the addition of a solution of glycine (5.6 mg, 74.6 µmol) in water (560 µl). A 0.5 % aqueous solution of ammonium acetate (34 ml) was added, and the resulting mixture eluted onto a Varian 5g C8 Mega Bond Elut®, the immobilised compound washed with 5% aqueous acetonitril (15 ml), and finally liberated from the cartridge by elution with TFA (25 ml). The solvent was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (3.1 mg, 24 %) was isolated, and the product was analysed by PDMS.

### Example 19

### Synthesis of Arg^{26,34},Lys¹⁸ (N^{ε}-(γ-glutamyl(N^{α}-hexadecanoyl))) GLP-1 (7-37)-OH

To a mixture of Arg^{26,34}, Lys¹⁸GLP-1 (7-37)-OH (11.7 mg, 3.4 µmol), EDPA (12.2 mg, 94.6 µmol), NMP (1.6 ml) and water (0.8 ml) was added a solution of Pal-Glu(ONSu)-OBu^{t} (5.5 mg, 10.2 µmol) in NMP (137 µl). The reaction mixture was gently shaken for 5 min. at room temperature, and then allowed to stand for an additional 90 min. at room temperature. The reaction was quenched by the addition of a solution of glycine (5.6 mg, 74.6 µmol) in water (560 µl). A 0.5 % aqueous solution of ammonium acetate (34 ml) was added, and the resulting mixture eluted onto a Varian 5g C8 Mega Bond Elut®, the immobilised compound washed with 5% aqueous acetonitril (25 ml), and finally liberated from the cartridge by elution with TFA (25 ml). The solvent was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (1.9 mg, 15 %) was isolated, and the product was analysed by PDMS.

### Example 20

### Synthesis of Arg³⁴,Lys²⁶ (N^{ε}-(octanoyl)) GLP-1 (7-37)-OH.

To a mixture of Arg³⁴ GLP-1 (7-37)-OH (41.1 mg, 12.2 µmol), EDPA (44 mg, 341 µmol), NMP (5.76 ml) and water (2.88 ml) was added a solution of Cap-ONSu (8.8 mg, 36.5 µmol, prepared as described in example 11, in NMP (106 µl). The reaction mixture was gently shaken for 5 min. at room temperature, and then allowed to stand for an additional 115 min. at room temperature. The reaction was quenched by the addition of a solution of glycine (20 mg, 268 µmol) in water (200 µl). The solvent was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (18.8 mg, 44 %) was isolated, and the product was analysed by PDMS.

### Example 21

### Synthesis of Arg³⁴,Lys²⁶ (N^{ε}-(dodecanoyl)) GLP-1 (7-37)-OH.

To a mixture of Arg³⁴ GLP-1 (7-37)-OH (41.1 mg, 12.2 µmol), EDPA (44 mg, 341 µmol), NMP (5.76 ml) and water (2.88 ml) was added a solution of Lau-ONSu (8.8 mg, 36.5 µmol, prepared in a similar manner as described for Cap-ONSu in example 11), in NMP (271 µl). The reaction mixture was gently shaken for 5 min. at room temperature, and then allowed to stand for an additional 100 min. at room temperature. The reaction was quenched by the addition of a solution of glycine (20.1 mg, 268 µmol) in water (200 µl). The solvent was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (18 mg, 42 %) was isolated, and the product was analysed by PDMS.

### Example 22

### Synthesis of Pal-GABA-ONSu.

A mixture of Pal-ONSu (3 g, 8.48 mmol), γ-aminobutyric acid (0.87 g, 8.48 mmol) in DMF (200 ml) was stirred at room temperature for 60 h. The reaction mixture was filtered and the filtrate was added drop wise to 10% aqueous citric acid (500 ml). The precipitated N-acylated intermediate was collected and dried *in vacuo*. To a suspension of the dried intermediate in DMF (35 ml) was added a solution of DCC (1.45 g, 7.0 mmol) in dichloromethane (20 ml). The resulting mixture was stirred at room temperature for 20 h, and then filtered. The solvent was removed *in vacuo* to give a solid residue. The residue was recrystallised from a mixture of n-heptane (50 ml) and 2-propanol (2.5 ml) to give the title compound (2.5 g, 75 %).

### Example 23

### Synthesis of Arg³⁴,Lys²⁶ (N^{ε}-(γ-aminobutyroyl(N^{γ}-hexadecanoyl))) GLP-1 (7-37)-OH.

To a mixture of Arg³⁴, Lys²⁶GLP-1 (7-37)-OH (41.1 mg, 12.2 µmol), EDPA (44 mg, 341 µmol), NMP (5.76 ml) and water (2.88 ml) was added a solution of Pal-GABA-ONSu (16 mg, 36.5 µmol, prepared as described in example 22) in NMP (400 µl). The reaction mixture was gently shaken for 5 min. at room temperature, and then allowed to stand for an additional 100 min. at room temperature. The reaction was quenched by the addition of a solution of glycine (20 mg, 268 µmol) in water (200 µl). The solvent was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (15.8 mg, 35 %) was isolated, and the product was analysed by PDMS.

### Example 24

### Synthesis of N^{α}-hexadecanoyl-D-glutamic acid α-t-butyl ester-γ-2,5-dioxopyrrolidin-1-yl ester.

A mixture of Pal-ONSu (6.64 g, 18.8 mmol), D-glutamic acid α-tert-butyl ester (4.5 g, 18.8 mmol) and EDPA (4.85 g, 37.5 mmol) in DMF (538 ml) was stirred at room temperature for 60 h. The solvent was removed and the residue dissolved in ethyl acetate (175 ml). The resulting solution was extracted with 10% aqueous citric acid (2x125 ml), and the organic phase concentrated *in vacuo*. The residue was dissolved in DMF (60 ml), and the resulting mixture slowly added to 10% aqueous citric acid (500 ml). The precipitated compound was collected and dried *in vacuo,* to give the crude N-acylated glutamic acid intermediate. The crude intermediate was dissolved in DMF (35 ml), and a solution of DCC (3.5 g, 17 mmol) in dichloromethane (70 ml) was added. The resulting mixture was stirred at room temperature for 20 h, and then filtered. The filtrate was concentrated *in vacuo*, and the solid residue recrystallised from a mixture of n-heptane (75 ml) and 2-propanol (5 ml), to give the title compound (5.2 g, 50 %)

### Example 25

### Synthesis of Arg³⁴,Lys²⁶ (N^{ε}-(γ-D-glutamyl(N^{α}-hexadecanoyl))) GLP-1 (7-37)-OH.

To a mixture of Arg³⁴, Lys²⁶ GLP-1 (7-37)-OH (41.1 mg, 12.2 µmol), EDPA (44 mg, 341 µmol), NMP (5.76 ml) and water (2.88 ml) was added a solution of N^{α}-hexadecanoyl-D-glutamic acid α-t-butyl ester-γ-2,5-dioxopyrrolidin-1-yl ester (19.7 mg, 36.5 µmol) in NMP (491 µl). The reaction mixture was gently shaken for 5 min. at room temperature, and then allowed to stand for an additional 95 min. at room temperature. The reaction was quenched by the addition of a solution of glycine (20 mg, 268 µmol) in water (2 ml). A 0.5 % aqueous solution of ammonium acetate (120 ml) was added, and the resulting mixture divided into to equal portions, and each portion eluted onto a Varian 5g C8 Mega Bond Elut®, the immobilised compound washed with 5% aqueous acetonitril (25 ml), and finally liberated from the cartridge by elution with TFA (25 ml). The combined eluates were concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (10.5 mg, 23 %) was isolated, and the product was analysed by PDMS.

### Example 26

### Synthesis of Lys³⁴ (N^{ε}-(γ-glutamyl(N^{α}-tetradecanoyl))) GLP-1 (7-37).

To a mixture of GLP-1 (7-37)-OH (33.6 mg, 8.9 µmol), EDPA (32.4 mg, 250 µmol), NMP (2.1 ml) and water (2.1 ml) was added a solution of Myr-Glu(ONSu)-OBu^{t} (9.1 mg, 17.9 µmol), prepared as described in PCT application no. PCT/DK97/00340, in NMP (228 µl). The reaction mixture was gently shaken for 5 min., and then allowed to stand for an additional 80 min. at room temperature. The reaction was quenched by the addition of a solution of glycine (14.8 mg, 197 µmol) in water (1.47 ml). A 0.5% aqueous solution of ammonium acetate (100 ml) was added, and the resulting mixture divided into two equal portions, and each portion eluted onto a Varian 5g C8 Mega Bond Elut®, the immobilised compound washed with 5% aqueous acetonitril (2x25 ml), and finally liberated from the cartridge by elution with TFA (2x25 ml). The combined eluates were concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (0.19 mg, 0.6%) was isolated, and the product was analysed by PDMS. The m/z value for the protonated molecular ion was found to be 3693 ± 3. The resulting molecular weight is thus 3692 ± 3 amu (theoretical value 3695 amu).

### Example 27

### Synthesis of Arg^{26,34},Lys⁸(N^{ε}-(γ-glutamyl(N^{α}-hexadecanoyl))) GLP-1 (7-37).

To a mixture of Arg^{26,34}, Lys⁸-GLP-1 (7-37)-OH (10.3 mg, 3 µmol), EDPA (10.8 mg, 83 µmol), NMP (1.44 ml) and water (0.72 ml) was added a solution of Pal-Glu(ONSu)-OBu^{t} (4.8 mg, 8.9 µmol), prepared as described in PCT application no. PCT/DK97/00340, in NMP (120 µl). The reaction mixture was gently shaken for 5 min., and then allowed to stand for an additional 70 min. at room temperature. The reaction was quenched by the addition of a solution of glycine (4.9 mg, 65.3 µmol) in water (490 µl). A 0.5% aqueous solution of ammonium acetate (30 ml) was added, and the resulting mixture eluted onto a Varian 5g C8 Mega Bond Elut®, the immobilised compound washed with 5% aqueous acetonitril (25 ml), and finally liberated from the cartridge by elution with TFA (25 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (3.2 mg, 28%) was isolated, and the product was analysed by PDMS. The m/z value for the protonated molecular ion was found to be 3836 ± 3. The resulting molecular weight is thus 3835 ± 3 AMU (theoretical value 3836 AMU).

### Example 28

### Synthesis of Lau-Glu(ONSu)-OBu^{t}.

To a solution of H-Glu-OBu^{t} (3 g, 15 mmol) in DMF (344 ml) was added EDPA (2.58 ml, 15 mmol) and a solution of Lau-ONSu (4.5 g, 15 mmol), prepared in a similar manner as described for Cap-ONSu in example 11, in DMF (74 ml). The resulting mixture was stirred at ambient temperature for 18 h, and the solvent removed *in vacuo*. The oily residue was partitioned between ethyl acetate (150 ml) and 5% aqueous citric acid (250 ml). The organic phase was concentrated *in vacuo.* The residue was dissolved in DMF (40 ml) and the solution added drop by drop to a 10% aqueous citric acid solution (350 ml). The precipitated product was collected, washed with water and dried *in vacuo* for 18 h to give the intermediate free acid. To solution of the free acid intermediate in DMF (25 ml) was added N-hydroxysuccinimide (1.7 g, 14.8 mmol) and a solution of N-(3-dimethylaminopropyl)-N'-ethylcarbodiimide (2.58 g, 13.5 mmol) in dichloromethane (52 ml). The resulting mixture was stirred at room temperature for 18 h, and the solvents removed *in vacuo*. The oily residue was partitioned between dichloromethane (80 ml) and water (80 ml). The organic phase was washed with 5% aqueous citric acid, dried (MgSO₄), and concentrated *in vacuo* to a solid. The solid residue was crystallised from a mixture of n-heptane (77 ml) and 2-propanol (50 ml), and finally recrystallised from n-heptane (76 ml) to give the title compound (2.96 g, 46%).

### Example 29

### Synthesis of Arg³⁴,Lys²⁶(N^{ε}-(γ-glutamyl(N^{α}-dodecanoyl))) GLP-1 (7-37).

To a mixture of Arg³⁴-GLP-1 (7-37)-OH (20.6 mg, 6.1 µmol), EDPA (22 mg, 171 µmol), NMP (2.88 ml) and water (1.44 ml) was added a solution Lau-Glu(ONSu)-OBu^{t} (10.2 mg, 21.2 µmol), prepared as described in example 28, in NMP (255 µl). The reaction mixture was gently shaken for 5 min., and then allowed to stand for an additional 75 min. at room temperature. The reaction was quenched by the addition of a solution of glycine (10 mg, 134 µmol) in water (100 µl). A 0.5% aqueous solution of ammonium acetate (61 ml) was added, and the resulting mixture eluted onto a Varian 5g C8 Mega Bond Elut®, the immobilised compound washed with 5% aqueous acetonitril (25 ml), and finally liberated from the cartridge by elution with TFA (25 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (8.2 mg, 36%) was isolated, and the product was analysed by PDMS. The m/z value for the protonated molecular ion was found to be 3693 ± 3. The resulting molecular weight is 3692 ± 3 AMU (theoretical value 3693 AMU).

### Example 30

### Synthesis of Lau-β-Ala-ONSu.

To a solution of Lau-ONSu (4.25 g, 14.3 mmol), prepared in a similar manner to in DMF (400 ml) was added EDPA (1.84 g, 14.3 mmol) and β-alanine (1.27 g, 14.3 mmol). The resulting mixture was stirred at ambient temperature for 18 h. Water (250 ml) and DMF (50 ml) were added and the solution stirred for 1 h at room temperature. The solvents were removed *in vacuo* to give a solid. The solid residue was dissolved in DMF (50 ml) and the solution added drop by drop to a 5% aqueous solution of citric acid (200 ml). The precipitate collected, washed with water (50 ml) and dried *in vacuo* to give the title compound (3.6 g, 93%).

### Example 31

### Synthesis of Pal-β-Ala-ONSu.

To a solution of Pal-ONSu (4.25 g, 14.3 mmol) in DMF (400 ml) was added EDPA (1.84 g, 14.3 mmol) and β-alanine (1.27 g, 14.3 mmol). The resulting mixture was stirred at ambient temperature for 18 h. Water (250 ml) and DMF (50 ml) were added and the solution stirred for 1 h at room temperature. The solvents were removed *in vacuo* to give a solid. The solid residue was dissolved in DMF (50 ml) and the solution added drop by drop to a 5% aqueous solution of citric acid (200 ml). The precipitate collected, washed with water (50 ml) and dried *in vacuo* to give the title compound (3.6 g, 93%).

### Example 32

### Synthesis of Myr-GABA-ONSu.

To a solution of Myr-ONSu (4 g, 12.3 mmol) in DMF (350 ml) was added EDPA (1.58 g, 12.3 mmol) and γ-aminobutyric acid (1.26 g, 12.3 mmol). The resulting mixture was stirred at ambient temperature for 18 h. Water (50 ml) was added and the solution stirred for 1 h at room temperature. The solvents were removed *in vacuo* to give a solid. The solid residue was dissolved in DMF (75 ml) and the solution added drop by drop to a 5% aqueous solution of citric acid (250 ml). The precipitate collected, washed with water (100 ml) and dried *in vacuo* to give the free acid intermediate (3.65 g, 95%). To a solution of the free acid intermediate (3 g, 9.6 mmol), N-hydroxysuccinimide (1.65 g, 14.4 mmol) and N-(3-dimethylaminopropyl)-N'-ethylcarbodiimide hydrochloride (3.67 g, 19.1 mmol) in DMF (330 ml) was stirred for 18 h at room temperature, and the solvent removed *in vacuo* to give a solid. The solid residue was dissolved in dichloromethane (100 ml) and washed with brine (100 ml). The organic phase was dried (MgSO₄) and concentrated *in vacuo* to give a solid. The solid residue was recrystallised from n-heptane (75 ml) to give the title compound (2.8 g, 71%).

### Example 33

### Synthesis of Pal-β-Ala-ONSu.

To a solution of Pal-ONSu (0.9 g, 2.8 mmol) in DMF (100 ml) were added N-hydroxysuccinimide (0.35 g, 3 mmol) and N-(3-dimethylaminopropyl)-N'-ethylcarbodiimide (0.79 g, 4.1 mmol). The resulting mixture was stirred at ambient temperature for 40h, and the solvent removed *in vacuo*. The solid residue was partitioned between water (50 ml) and dichloromethane (50 ml). The organic phase was separated, dried (MgSO₄) and the solvent removed *in vacuo* to give the title compound (1.1 g, 94%).

### Example 34

### Synthesis of Arg³⁴,Lys²⁶(N^{ε}-(β-alanyl(N^{α}-hexadecanoyl))) GLP-1 (7-37).

To a mixture of Arg³⁴-GLP-1 (7-37)-OH (19.2 mg, 5.7 µmol), EDPA (20.5 mg, 159 µmol), NMP (2.7 ml) and water (1.35 ml) was added a solution Pal-β-Ala-ONSu (7.2 mg, 17 µmol), prepared as described in example 33, in NMP (181 µl). The reaction mixture was gently shaken for 5 min., and then allowed to stand for an additional 90 min. at room temperature. The reaction was quenched by the addition of a solution of glycine (9.3 mg, 125 µmol) in water (93 µl). The reaction mixture was purified by column chromatography using a cyanopropyl column (Zorbax 300SB-CN) and a standard acetonitril/TFA system. The column was heated to 65°C and the acetonitril gradient was 0-100% in 60 minutes. The title compound (11.6 mg, 55%) was isolated, and the product was analysed by PDMS. The m/z value for the protonated molecular ion was found to be 3694 ± 3. The resulting molecular weight is thus 3693 ± 3 AMU (theoretical value 3693 AMU).

### Example 35

### Synthesis of Pal-Glu(OBu^{t})-ONSu.

To a solution of H-Glu(OH)-OBu^{t} (2.7 g, 11.3 mmol) and Pal-ONSu (3.98 g, 11.3 mmol) in DMF (300 ml) was added EDPA (3.2 g, 24.8 mmol). The resulting mixture was stirred at ambient temperature for 18h, and the solvent concentrated *in vacuo* to give an oil. The oily residue was dissolved in DMF (60 ml) and the solution added drop by drop to a 10% aqueous solution of citric acid (300 ml) whereby a precipitate was formed. The precipitate was collected, washed with cold water (25 ml), and dried *in vacuo* to give free acid intermediate (4.44 g, 89%). The free acid intermediate (4 g, 9.1 mmol) was dissolved in DMF (50 ml) and N-hydroxysuccinimide (1.15 g, 10 mmol) and N-(3-dimethylaminopropyl)-N'-ethylcarbodiimide hydrochloride (2.6 g, 13.6 mmol) were added. The resulting mixture was stirred at room temperature for 60h, the solvent concentrated *in vacuo* to give the crude title compound (8.2 g)

### Example 36

### Synthesis of Arg³⁴,Lys²⁶(N^{ε}-(α-glutamyl(N^{α}-hexadecanoyl))) GLP-1 (7-37).

To a mixture of Arg³⁴-GLP-1 (7-37)-OH (25.6 mg, 7.6 µmol), EDPA (27.4 mg, 212 µmol), NMP (3.5 ml) and water (1.75 ml) was added a solution of Pal-Glu(OBu^{t})-ONSu (12.2 mg, 22.7 µmol), prepared as described in example 35, in NMP (305 µl). The reaction mixture was gently shaken for 5 min., and then allowed to stand for an additional 100 min. at room temperature. The reaction was quenched by the addition of a solution of glycine (12.5 mg, 168 µmol) in water (125 µl). A 0.5% aqueous solution of ammonium acetate (72.5 ml) was added, and the resulting mixture eluted onto a Varian 5g C8 Mega Bond Elut®, the immobilised compound washed with 5% aqueous acetonitril (25 ml), and finally liberated from the cartridge by elution with TFA (30 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (6.1 mg, 22%) was isolated, and the product was analysed by PDMS. The m/z value for the protonated molecular ion was found to be 3751 ± 3. The resulting molecular weight is thus 3750 ± 3 AMU (theoretical value 3751 AMU).

### Example 37

### Synthesis of Ste-GABA-ONSu.

To a solution of Ste-ONSu (3 g, 7.9 mmol) in DMF (270 ml) was added EDPA (1 g, 7.9 mmol) and a solution of γ-aminobutyric acid (0.81 g, 7.9 mmol) in water (40 ml). The resulting suspension was stirred at ambient temperature for 18 h, and then concentrated *in vacuo* to a final volume of 50 ml. The resulting suspension was added to a 5% aqueous solution of citric acid (500 ml) whereby a precipitate is formed. The precipitate was collected and washed with water (50 ml), and dried *in vacuo* for 4h to give the free acid intermediate (2.8 g, 97%). To a mixture of the free acid intermediate (2.6 g, 7 mmol), N-hydroxysuccinimide (1.21 g, 10.5 mmol) and N-(3-dimethylaminopropyl)-N'-ethylcarbodiimide hydrochloride (2.69 g, 14 mmol) in NMP (300 ml) was stirred for 70 h, and the solvent removed *in vacuo* to give a solid. The solid residue was dissolved in dichloromethane (100 ml) and washed with brine (2x100 ml). The organic phase was dried (MgSO₄) and concentrated *in vacuo* to give a solid. The solid residue was recrystallised from n-heptane (75 ml) to give the title compound (2.2 g, 67%).

### Example 38

### Synthesis of Pal-Isonip-ONSu.

To a suspension of 1-hexadecanoylbenzotriazole (3 g, 8.4 mmol), prepared as described in the literature (Kreutzberger; van der Goot, Arch.Pharm., 307, 1974), in DMF (350 ml) were added EDPA (1.08 g, 8.4 mmol) and a solution of piperidine-4-carboxylic acid in water ( 20 ml). The resulting suspension was stirred at room temperature for 12d, and then concentrated *in vacuo* to an oil. The oily residue was added drop by drop to a 5% aqueous solution of citric acid (300 ml) whereby a precipitate was formed. The precipitate was collected and washed with water (50 ml), dried *in vacuo* for 2 h to give the free acid intermediate (3 g, 97%). To a solution of the free acid intermediate (2.8 g, 7.6 mmol), N-hydroxysuccinimide (1.31 g, 11.4 mmol) in DMF (250 ml) was added N-(3-dimethylaminopropyl)-N'-ethylcarbodiimide hydrochloride (2.92 g, 15.2 mmol). The resulting mixture was stirred at ambient temperature for 18h, and the solvent removed *in vacuo* to give an oil. The oily residue was dissolved in dichloromethane (100 ml), washed with brine (50 ml), dried (MgSO₄) and concentrated *in vacuo* to give the crude title compound (4.1 g, quant.).

### Example 39

### Synthesis of Arg³⁴,Lys²⁶(N^{ε}-(piperidinyl-4-carbonyl(N-hexadecanoyl))) GLP-1 (7-37).

To a mixture of Arg³⁴-GLP-1 (7-37)-OH (25 mg, 7.4 µmol), EDPA (26.7 mg, 207 µmol), NMP (3.5 ml) and water (1.75 ml) was added a solution Pal-isonip-ONSu (13.7 mg, 30 µmol), prepared as described in example 38 in NMP (343 µl). The reaction mixture was gently shaken for 5 min., and then allowed to stand for an additional 90 min. at room temperature. The reaction was quenched by the addition of a solution of glycine (12.2 mg, 163 µmol) in water (122 µl). The reaction mixture was purified by column chromatography using a cyanopropyl column (Zorbax 300SB-CN) and a standard acetonitril/TFA system. The column was heated to 65°C and the acetonitril gradient was 0-100% in 60 minutes. The title compound (12 mg, 44%) was isolated, and the product was analysed by PDMS. The m/z value for the protonated molecular ion was found to be 3734 ± 3. The resulting molecular weight is thus 3733 ± 3 AMU (theoretical value 3733 AMU).

### Example 40

### Synthesis of Arg³⁴,Lys²⁶(N^{ε}-(γ-glutamyl(N^{α}-decanoyl))) GLP-1 (7-37).

To a mixture of Arg³⁴-GLP-1 (7-37)-OH (25 mg, 7.4 µmol), EDPA (26.7 mg, 207 µmol), NMP (3.5 ml) and water (1.75 ml) was added a solution of Cac-Glu(ONSu)-OBu^{t} (10 mg, 22.1 µmol) in NMP (252 µl). The reaction mixture was gently shaken for 5 min., and then allowed to stand for an additional 140 min. at room temperature. The reaction was quenched by the addition of a solution of glycine (12.2 mg, 162 µmol) in water (122 µl). A 0.5% aqueous solution of ammonium acetate (73 ml) was added, and the resulting mixture eluted onto a Varian 5g C8 Mega Bond Elut®, the immobilised compound washed with 5% aqueous acetonitril (25 ml), and finally liberated from the cartridge by elution with TFA (25 ml). The eluate was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (12.2 mg, 45%) was isolated, and the product was analysed by PDMS. The m/z value for the protonated molecular ion was found to be 3669.7 ± 3. The resulting molecular weight is thus 3668.7 ± 3 amu (theoretical value 3667 amu).

### Examples of pharmaceutical formulations

In the following "8 mM phosphate buffer of pH 7.4" is intended to mean: 4 mM NaH₂PO₄, 2H₂O and 4 mM Na₂HPO₄, 2H₂O;
pH adjusted to 7.4 (using Sodium Hydroxide and/or Hydrochloric acid).

In the following "Compound 1" is intended to mean: Arg³⁴,Lys²⁶(N^{ε}-(^{γ}-Glu(N^{α}-tetradecanoyl))) GLP-1 (7-37).

In the following ''Compound 2" is intended to mean: Arg³⁴,Lys²⁶(N^{ε}-(^{γ}-Glu(N^{α}-hexadecanoyl))) GLP-1 (7-37).

In the following "Compound 3" is intended to mean: Arg^{26,34},Lys³⁶(N^{ε}-(^{γ}-Glu(N^{α}-hexadecanoyl))) GLP-1 (7-36).

In the following "Compound 4" is intended to mean: Arg²⁶,Lys³⁴(N^{ε}-(^{γ}-Glu(N^{α}-hexadecanoyl))) GLP-1 (7-37).

In the following "Compound 5" is intended to mean: Gly⁸,Glu³⁷,Arg^{26,34},Lys³⁸(N^{ε}-(^{γ}-Glu(N^{α}-hexadecanoyl))) GLP-1 (7-38).

| **General example A** | |
|---|---|
| Compound | 2 - 7.5 mg/ml |
| Mannitol | 34-50 mg/ml |
| Phenol | 5 - 7.5 mg/ml |
| 8 mM phosphate buffer of pH 7.4 | |

Mannitol and phenol were dissolved in the phosphate buffer preadjusted to pH 7.4. Hereafter, the Compound were dissolved under slow stirring. The pH were adjusted to 7.4 using Sodium Hydroxide and/or Hydrochloric Acid. Finally, the formulation were sterilised by filtration through an appropriate filter.

The following specific formulations were produced using the procedure under general example A:

| Example A1 | |
|---|---|
| Compound 1 | 2.0 mg/ml |
| Mannitol | 38 mg/ml |
| Phenol | 5 mg/ml |
| 8 mM phosphate buffer of pH 7.4 ad 100 ml | |

| Example A2 | |
|---|---|
| Compound 1 | 5 mg/ml |
| Mannitol | 36.9 mg/ml |
| Phenol | 5 mg/ml |
| 8 mM phosphate buffer of pH 7.4 ad 100 ml | |

| Example A3 | |
|---|---|
| Compound 1 | 7.5 mg/ml |
| Mannitol | 34 mg/ml |
| Phenol | 7.5 mg/ml |
| 8 mM phosphate buffer of pH 7.4 ad 100 ml | |

| Example A4 | |
|---|---|
| Compound 2 | 2.0 mg/ml |
| Mannitol | 38 mg/ml |
| Phenol | 5 mg/ml |
| 8 mM phosphate buffer of pH 7.4 ad 100 ml | |

| Example A5 | |
|---|---|
| Compound 2 | 5 mg/ml |
| Mannitol | 36.9 mg/ml |
| Phenol | 5 mg/ml |
| 8 mM phosphate buffer of pH 7.4 ad 100 ml | |

| Example A6 | |
|---|---|
| Compound 2 | 7.5 mg/ml |
| Mannitol | 34 mg/ml |
| Phenol | 7.5 mg/ml |
| 8 mM phosphate buffer of pH 7.4 ad 100 ml | |

| Example A7 | |
|---|---|
| Compound 3 | 2.0 mg/ml |
| Mannitol | 38 mg/ml |
| Phenol | 5 mg/ml |
| 8 mM phosphate buffer of pH 7.4 ad 100 ml | |

| Example A8 | |
|---|---|
| Compound 3 | 5 mg/ml |
| Mannitol | 36.9 mg/ml |
| Phenol | 5 mg/ml |
| 8 mM phosphate buffer of pH 7.4 ad 100 ml | |

| Example A9 | |
|---|---|
| Compound 3 | 7.5 mg/ml |
| Mannitol | 34 mg/ml |
| Phenol | 7.5 mg/ml |
| 8 mM phosphate buffer of pH 7.4 ad 100 ml | |

| Example A10 | |
|---|---|
| Compound 4 | 2.0 mg/ml |
| Mannitol | 38 mg/ml |
| Phenol | 5 mg/ml |
| 8 mM phosphate buffer of pH 7.4 ad 100 ml | |

| Example A11 | |
|---|---|
| Compound 4 | 5 mg/ml |
| Mannitol | 36.9 mg/ml |
| Phenol | 5 mg/ml |
| 8 mM phosphate buffer of pH 7.4 ad 100 ml | |

| Example A12 | |
|---|---|
| Compound 4 | 7.5 mg/ml |
| Mannitol | 34 mg/ml |
| Phenol | 7.5 mg/ml |
| 8 mM phosphate buffer of pH 7.4 ad 100 ml | |

| **General example B** | |
|---|---|
| Compound | 2 - 7.5 mg/ml |
| Mannitol | 19-25 mg/ml |
| Benzyl Alcohol | 14-18 mg/ml |
| 8 mM phosphate buffer of pH 7.4 | |

Mannitol and benzyl alcohol were dissolved in the phosphate buffer preadjusted to pH 7.4. Hereafter, the Compound were dissolved under slow stirring. The pH were adjusted to 7.4 using Sodium Hydroxide and/or Hydrochloric Acid. Finally, the formulation were sterilised by filtration through an appropriate filter.

The following specific formulations were produced using the procedure under general example B:

| Example B1 | |
|---|---|
| Compound 1 | 2.0 mg/ml |
| Mannitol | 25 mg/ml |
| Benzyl alcohol | 14 mg/ml |
| 8 mM phosphate buffer of pH 7.4 ad 100 ml | |

| Example B2 | |
|---|---|
| Compound 1 | 7.5 mg/ml |
| Mannitol | 19 mg/ml |
| Benzyl alcohol | 18 mg/ml |
| 8 mM phosphate buffer of pH 7.4 ad 100 ml | |

| Example B3 | |
|---|---|
| Compound 2 | 2.0 mg/ml |
| Mannitol | 25 mg/ml |
| Benzyl alcohol | 14 mg/ml |
| 8 mM phosphate buffer of pH 7.4 ad 100 ml | |

| Example B4 | |
|---|---|
| Compound 2 | 7.5 mg/ml |
| Mannitol | 19 mg/ml |
| Benzyl alcohol | 18 mg/ml |
| 8 mM phosphate buffer of pH 7.4 ad 100 ml | |

| Example B5 | |
|---|---|
| Compound 3 | 2.0 mg/ml |
| Mannitol | 25 mg/ml |
| Benzyl alcohol | 14 mg/ml |
| 8 mM phosphate buffer of pH 7.4 ad 100 ml | |

| Example B6 | |
|---|---|
| Compound 3 | 7.5 mg/ml |
| Mannitol | 19 mg/ml |
| Benzyl alcohol | 18 mg/ml |
| 8 mM phosphate buffer of pH 7.4 ad 100 ml | |

| Example B7 | |
|---|---|
| Compound 5 | 2.0 mg/ml |
| Mannitol | 25 mg/ml |
| Benzyl alcohol | 14 mg/ml |
| 8 mM phosphate buffer of pH 7.4 ad 100 ml | |

| Example B8 | |
|---|---|
| Compound 5 | 7.5 mg/ml |
| Mannitol | 19 mg/ml |
| Benzyl alcohol | 18 mg/ml |
| 8 mM phosphate buffer of pH 7.4 ad 100 ml | |

| **General example C** | |
|---|---|
| Compound | 2 - 7.5 mg/ml |
| Mannitol | 42-44 mg/ml |
| Metacresol | 2.5-4.0 mg/ml |
| 8 mM phosphate buffer of pH 7.4 | |

Mannitol and metacresol were dissolved in the phosphate buffer preadjusted to pH 7.4. Hereafter, the Compound were dissolved under slow stirring. The pH were adjusted to 7.4 using Sodium Hydroxide and/or Hydrochloric Acid. Finally, the formulation were sterilised by filtration through an appropriate filter.

The following specific formulations were produced using the procedure under general example C:

| Example C1 | |
|---|---|
| Compound 1 | 2.0 mg/ml |
| Mannitol | 44 mg/ml |
| Metacresol | 2.5 mg/ml |
| 8 mM phosphate buffer of pH 7.4 ad 100 ml | |

| Example C2 | |
|---|---|
| Compound 1 | 7.5 mg/ml |
| Mannitol | 42 mg/ml |
| Metacresol | 4 mg/ml |
| 8 mM phosphate buffer of pH 7.4 ad 100 ml | |

| Example C3 | |
|---|---|
| Compound 2 | 2.0 mg/ml |
| Mannttol | 44 mg/ml |
| Metacresol | 2.5 mg/ml |
| 8 mM phosphate buffer of pH 7.4 ad 100 ml | |

| Example C4 | |
|---|---|
| Compound 2 | 7.5 mg/ml |
| Mannitol | 42 mg/ml |
| Metacresol | 4 mg/ml |
| 8 mM phosphate buffer of pH 7.4 ad 100 ml | |

| Example C5 | |
|---|---|
| Compound 3 | 2.0 mg/ml |
| Mannitol | 44 mg/ml |
| Metacresol | 2.5 mg/ml |
| 8 mM phosphate buffer of pH 7.4 ad 100 ml | |

| Example C6 | |
|---|---|
| Compound 3 | 7.5 mg/ml |
| Mannitol | 42 mg/ml |
| Metacresol | 4 mg/ml |
| 8 mM phosphate buffer of pH 7.4 ad 100 ml | |

| Example C7 | |
|---|---|
| Compound 5 | 2.0 mg/ml |
| Mannitol | 44 mg/ml |
| Metacresol | 2.5 mg/ml |
| 8 mM phosphate buffer of pH 7.4 ad 100 ml | |

| Example C8 | |
|---|---|
| Compound 5 | 7.5 mg/ml |
| Mannitol | 42 mg/ml |
| Metacresol | 4 mg/ml |
| 8 mM phosphate buffer of pH 7.4 ad 100 ml | |

## Claims

1. A pharmaceutical composition comprising a GLP-1 derivative which has a helix content as measured by CD at 222 nm in H₂O at 22 ± 2°C exceeding 25%, preferably in the range of 25% to 50%, at a peptide concentration of about 10 µM, wherein the concentration of the GLP-1 derivative is not less than 0.5 mg/ml, and a preservative.

2. A pharmaceutical composition according to claim 1, wherein the concentration of GLP-1 derivative is not less than 5 mg/ml, more preferred not less than 10 mg/ml and, preferably, not more than 100 mg/ml.

3. A pharmaceutical composition according to claim 1 or 2, comprising a GLP-1 derivative wherein at least one amino acid residue of the parent peptide has a lipophilic substituent attached.

4. A pharmaceutical composition according to claim 3, comprising a GLP-1 derivative having a lipophilic substituent which is attached to any one of the amino acid residues in position 18-38, preferably 26-34.

5. A pharmaceutical composition according to any one of the preceding claims, further comprising a pharmaceutically acceptable vehide or carrier.

6. A pharmaceutical composition according to any one of the preceding daims, further comprising an isotonic agent, preferably selected from the group consisting of sodium chloride, mannitol and glycerol.

7. A pharmaceutical composition according to any one of the preceding claims, wherein the preservative is selected from the group consisting of phenol, m-cresol, methyl p-hydroxybenzoate, butyl p-hydroxybenzoate and benzyl alcohol.

8. A pharmaceutical composition according to any one of the preceding claims, further comprising a buffer, preferably selected from the group consisting of sodium acetate, citrate, glycylglycine, histidine, 2-phenylethanol and sodium phosphate.

9. A pharmaceutical composition according to any one of the preceding claims, further comprising a surfactant capable of improving the solubility and/or the stability of the GLP-1 derivative, preferable selected from poloxymer 188, tween 20 and tween 80.

10. A pharmaceutical composition according to according to any one of the preceding claims, comprising a GLP-1 derivative wherein the lipophilic substituent comprises from 4 to 40 carbon atoms, preferably from 8 to 25 carbon atoms.

11. A pharmaceutical composition according to according to any one of the preceding claims, comprising a GLP-1 derivative wherein a lipophilic substituent is attached to an amino acid residue in such a way that a carboxyl group of the lipophilic substituent forms an amide bond with an amino group of the amino add residue.

12. A pharmaceutical composition according to any one of the claims 1-11, comprising a GLP-1 derivative wherein a lipophilic substituent is attached to an amino add residue in such a way that an amino group of the lipophilic substituent forms an amide bond with a carboxyl group of the amino add residue.

13. A pharmaceutical composition according to any one of the preceding claims, comprising a GLP-1 derivative wherein the lipophilic substituent is attached to the parent peptide by means of a spacer.

14. A pharmaceutical composition according to claim 13, wherein the spacer is an unbranched alkane α,ω-dicarboxylic acid group having from 1 to 7 methylene groups, preferably two methylene groups, which form a bridge between an amino group of the parent peptide and an amino group of the lipophilic substituent.

15. A pharmaceutical composition according to claim 13, wherein the spacer is an amino add residue except Cys, or a dipeptide such as Gly-Lys or any unbranched alkane α,ω-aminoadd having from 1 to 7 methylene groups, preferably 2-4 methylene groups, which form a bridge between an amino group of the parent peptide and an amino group of the lipophilic substituent.

16. A pharmaceutical composition according to any one of the preceding daims, comprising a GLP-1 derivative wherein the lipophilic substituent comprises a partially or completely hydrogenated cydopentanophenathrene skeleton.

17. A pharmaceutical composition according to any one of claims 3 to 15, wherein the lipophilic substituent is a straight-chain or branched alkyl group.

18. A pharmaceutical composition according to any one of daims 3 to 15, comprising a GLP-1 derivative wherein the lipophilic substituent is the acyl group of a straight-chain or branched fatty acid.

19. A pharmaceutical composition according to claim 18 wherein the acyl group is selected from the group comprising CH₃(CH₂)ₙCO-, wherein n is 4 to 38, preferably CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

20. A pharmaceutical composition according to any one of claims 3 to 15, comprising a GLP-1 derivative wherein the lipophilic substituent is an acyl group of a straight-chain or branched alkane α,ω-dicarboxylic acid.

21. A pharmaceutical composition according to claim 20 wherein the acyl group is selected from the group comprising HOOC(CH₂)ₘCO-, wherein m is from 4 to 38, preferably from 4 to 24, more preferred selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

22. A pharmaceutical composition according to any one of claims 3 to 15, comprising a GLP-1 derivative wherein the lipophilic substituent is a group of the formula CH₃(CH₂)ₚ((CH₂)_{q}COOH)CHNH-CO(CH₂)₂CO-, wherein p and q are integers and p+q is an integer of from 8 to 33, preferably from 12 to 28.

23. A pharmaceutical composition according to any one of claims 3 to 15, comprising a GLP-1 derivative, wherein the lipophilic substituent is a group of the formula CH₃(CH₂)ᵣCO-NHCH(COOH)(CH₂)₂CO-, wherein r is an integer of from 10 to 24.

24. A pharmaceutical composition according to any one of claims 3 to 15, comprising a GLP-1 derivative, wherein the lipophilic substituent is a group of the formula CH₃(CH₂)₆CO-NHCH((CH₂)₂COOH)CO-, wherein s is an integer of from 8 to 24.

25. A pharmaceutical composition according to any one of claims 3 to 15, comprising a GLP-1 derivative, wherein the lipophilic substituent is a group of the formula -NHCH(COOH)(CH₂)₄NH-CO(CH₂)ᵤCH₃, wherein u is an integer of from 8 to 18.

26. A pharmaceutical composition according to any one of claims 3 to 15, comprising a GLP-1 derivative, wherein the lipophilic substituent is a group of the formula -NHCH(COOH)(CH₂)₄NH-COCH((CH₂)₂COOH)NH-CO(CH₂)_{w}CH₃, wherein w is an integer of from 10 to 16.

27. A pharmaceutical composition according to any one of claims 3 to 15, comprising a GLP-1 derivative, wherein the lipophilic substituent is a group of the formula - NHCH(COOH)(CH₂)₄NH-CO(CH₂)₂CH(COOH)NH-CO(CH₂)ₓCH₃, wherein x is an integer of from 10 to 16.

28. A pharmaceutical composition according to any one of claims 3 to 15, comprising a GLP-1 derivative wherein the lipophilic substituent is a group of the formula -NHCH(COOH)(CH₂)₄NH-CO(CH₂)₂CH(COOH)NH-CO(CH₂)_{y}CH₃, wherein y is zero or an integer of from 1 to 22.

29. A pharmaceutical composition according to any one of the preceding daims, comprising a GLP-1 derivative wherein the parent peptide is GLP-1(A-B) wherein A is an integer from 1 to 7 and B is an integer from 38 to 45, or an analogue thereof.

30. A pharmaceutical composition according to claim 29, wherein the parent peptide Is selected from the group comprising GLP-1(7-35); GLP-1 (7-36); GLP-1(7-36)amide; GLP-1(7-37); GLP-1(7-38); GLP-1(7-39); GLP-1 (7-40) and GLP-1(7-41); and analogues thereof.

31. A pharmaceutical composition according to claim 29, wherein the parent peptide is selected from the group comprising GLP-1(1-35); GLP-1(1-36); GLP-1(1-36)amide; GLP-1(1-37); GLP-1(1-38); GLP-1(1-39); GLP-1(1-40); GLP-1(1-41); and an analogues thereof.

32. A pharmaceutical composition according to any of the preceding claims comprising a GLP-1 derivative wherein a total of up to fifteen, preferably up to ten, more preferably up to six, amino acid residues have been exchanged with any α-amino acid residue which can be coded for by the genetic code.

33. A pharmaceutical composition according to any of the preceding claims, comprising a GLP-1 derivative wherein the parent peptide is selected from the group comprising Arg²⁶-GLP-1(7-37); Arg³⁴-GLP-1(7-37); Lys³⁶-GLP-1(7-37); Arg^{26,34}Lys³⁶-GLP-1(7-37); Arg^{26,34}Lys³⁶GLP-1(7-38); Arg^{26,34}Lys³⁹-GLP-1(7-39); Arg^{26,34}Lys⁴⁰-GLP-1(7-40); Arg²⁶Lys³⁶-GLP-1(7-37); Arg³⁴Lys³⁶-GLP-1(7-37); Arg²⁶Lys³⁹-GLP-1(7-39); Arg³⁴Lys⁴⁰-GLP-1(7-40); Arg^{26,34}Lys^{36,39}-GLP-1(7-39); Arg^{26,34}Lys^{36,40}-GLP-1(7-40); Gly⁸Arg26-GLP-1(7-37); Gly⁸Arg³⁴-GLP-1(7-37); Gly⁸Lys³⁶-GLP-1(7-37); Gly⁸Arg^{26,34}Lys³⁶-GLP-1(7-37); Gly⁸Arg^{26,34}Lys³⁹-GLP-1(7-39); Gly⁸Arg^{26,34}Lys⁴⁰-GLP-1(7-40); Gly⁸Arg²⁶Lys³⁶-GLP-1(7-37); Gly⁸Arg³⁴Lys³⁶-GLP-1(7-37); Gly⁸Arg²⁶Lys³⁹-GLP-1(7-39); Gly⁸Arg³⁴Lys⁴⁰-GLP-1(7-40); Gly⁸Arg^{26,34}Lys^{36,39}-GLP-1(7-39) and Gly⁸Arg^{26,34}Lys^{36,40}-GLP-1(7-40).

34. A pharmaceutical composition according to any of the claims 1 to 32, comprising a GLP-1 derivative, wherein the parent peptide is selected from the group comprising Arg^{26,34}Lys³⁸GLP-1(7-38); Arg^{26,34}Lys³⁹GLP-1(7-39); Arg^{26,34}Lys⁴⁰GLP-1(7-40); Arg^{26,34}Lys⁴¹GLP-1(7-41); Arg^{26,34}Lys⁴²GLP-1(7-42); Arg^{26,34}Lys⁴³GLP-1(7-43); Arg^{26,34}Lys⁴⁴GLP-1(7-44); Arg^{26,34}Lys⁴⁵GLP-1(7-45); Arg^{26,34}Lys³⁸GLP-1(1-38); Arg^{26,34}Lys³⁹GLP-1(1-39); Arg^{26,34}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1(1-44); Arg^{26,34}Lys⁴⁵GLP-1(1-45); Arg^{26,34}Lys³⁸GLP-1(2-38); Arg^{26,34}Lys³⁹GLP-1(2-39); Arg^{26,34}Lys⁴⁰GLP-1(2-40); Arg^{26,34}Lys⁴¹GLP-1(2-41); Arg^{26,34}Lys⁴²GLP-1(2-42); Arg^{26,34}Lys⁴³GLP-1(2-43); Arg^{26,34}Lys⁴⁴GLP-1(2-44); Arg^{26,34}Lys⁴⁵GLP-1(2-45); Arg^{26,34}Lys³⁸GLP-1(3-38); Arg^{26,34}Lys³⁹GLP-1(3-39); Arg^{26,34}Lys⁴⁰GLP-1(3-40); Arg^{26,34}Lys⁴¹GLP-1(3-41); Arg^{26,34}Lys⁴²GLP-1(3-42); Arg^{26,34}Lys⁴³GLP-1(3-43); Arg^{26,34}Lys⁴⁴GLP-1(3-44); Arg^{26,34}Lys⁴⁵GLP-1(3-45); Arg^{26,34}Lys³⁸GLP-1(4-38); Arg^{26,34}Lys³⁹GLP-1(4-39); Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,34}Lys⁴⁵GLP-1(4-45); Arg^{26,34}Lys³⁸GLP-1(5-38); Arg^{26,34}Lys³⁹GLP-1(5-39); Arg^{26,34}Lys⁴⁰GLP-1(5-40); Arg^{26,34}Lys⁴¹GLP-1(5-41); Arg^{26,34}Lys⁴²GLP-1(5-42); Arg^{26,34}Lys⁴³GLP-1(5-43); Arg^{26,34}Lys⁴⁴GLP-1(5-44); Arg^{26,34}Lys⁴⁵GLP-1(5-45); Arg^{26,34}Lys³⁸GLP-1(6-38); Arg^{26,34}Lys³⁹GLP-1(6-39); Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁸GLP-1(1-38); Arg³⁴Lys³⁸GLP-1(1-38); Arg^{26,34}Lys^{36,38}GLP-1(1-38); Arg²⁶Lys³⁸GLP-1(7-38); Arg³⁴Lys³⁸GLP-1(7-38); Arg^{26,34}Lys^{36,38}GLP-1(7-38); Arg^{26,34}Lys³⁸GLP-1(7-38); Arg²⁶Lys³⁹GLP-1(1-39); Arg³⁴Lys³⁹GLP-1(1-39); Arg^{26,34}Lys^{36,39}GLP-1(1-39); Arg²⁶Lys³⁹GLP-1(7-39); Arg³⁴Lys³⁹GLP-1(7-39) and Arg^{26,34}Lys^{36,39}GLP-1(7-39).

35. A method for improving the solubility and/or stability of GLP-1 or a fragment or an analogue thereof, **characterised in that** a lipophilic substituent is introduced on any one of the amino acid residues of the parent peptide.

36. A method according to claim 35, wherein a lipophilic substituent is introduced on any one of the amino acid residues in position 18-38, preferably 26-34.

37. A method according to claim 35 or 36, wherein the lipophilic substituent comprises from 4 to 40 carbon atoms, preferably from 8 to 25 carbon atoms.

38. A method according to any one of claim 35 to 37, wherein the lipophilic substituent is the acyl group of a straight-chain or branched fatty acid.

39. A method according to any one of claim 36, wherein the acyl group is selected from the group comprising CH₃(CH₂)ₙCO-, wherein n is 4 to 38, preferably CH₃(CH₂)₈CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

40. A method according to any one of claims 35 to 39, wherein the GLP-1 is GLP-1(A-B) wherein A is an integer from 1 to 7 and B is an integer from 38 to 45, or an analogue thereof.

41. A method according to claim 40, wherein the GLP-1 is selected from the group comprising GLP-1(7-35); GLP-1(7-36); GLP-1(7-36)amide; GLP-1(7-37); GLP-1(7-38); GLP-1(7-39); GLP-1(7-40) and GLP-1(7-41); and analogues thereof.

42. A method according to claim 40, wherein the GLP-1 is selected from the group comprising GLP-1(1-35); GLP-1(1-36); GLP-1(1-36)amide; GLP-1(1-37); GLP-1(1-38); GLP-1(1-39); GLP-1(1-40); GLP-1(1-41); and an analogues thereof.

## Patentansprüche

1. Arzneimittel, umfassend ein GLP-Derivat mit einem Helix-Gehalt, gemessen mittels CD bei 222 nm in H₂O bei 22 ± 2°C, von über 25%, vorzugsweise im Bereich von 25% bis 50 %, bei einer Peptidkonzentration von etwa 10 µM, wobei die Konzentration des GLP-1-Derivats nicht weniger als 0,5 mg/ml beträgt, und ein Konservierungsmittel.

2. Arzneimittel nach Anspruch 1, wobei die Konzentration des GLP-1-Derivats nicht weniger als 5 mg/ml ist, stärker bevorzugt nicht weniger als 10 mg/ml und vorzugsweise nicht mehr als 100 mg/ml.

3. Arzneimittel nach Anspruch 1 oder 2, umfassend ein GLP-1-Derivat, wobei mindestens ein Aminosäurerest des Stammpeptids einen angehängten lipophilen Substituenten aufweist.

4. Arzneimittel nach Anspruch 3, umfassend ein GLP-1-Derivat mit einem lipophilen Substituenten, der an einem der Aminosäurereste in Position 18-38, vorzugsweise 26-34, angehängt wird.

5. Arzneimittel nach einem der vorhergehenden Ansprüche ferner umfassend ein pharmazeutisch verträgliches Bindemittel oder einen Träger.

6. Arzneimittel nach einem der vorhergehenden Ansprüche ferner umfassend ein isotonisches Mittel, das vorzugsweise aus der Gruppe, bestehend aus Natriumchlorid, Mannit und Glycerin, gewählt wird.

7. Arzneimittel nach einem der vorhergehenden Ansprüche, wobei das Konservierungsmittel aus der Gruppe, bestehend aus Phenol, m-Kresol, Methyl-p-Hydroxybenzoat, Butyl-p-Hydroxybenzoat und Benzylalkohol, gewählt wird.

8. Arzneimittel nach einem der vorhergehenden Ansprüche, ferner umfassend einen Puffer, der vorzugsweise aus der Gruppe, bestehend aus Natriumacetat, Citrat, Glycylglycin, Histidin, 2-Phenylethanol und Natriumphosphat, gewählt wird.

9. Arzneimittel nach einem der vorhergehenden Ansprüche, ferner umfassend ein oberflächeaktives Mittel, das in der Lage ist, die Löslichkeit und/oder die Stabilität des GLP-1-Derivats, das vorzugsweise aus Poloxymer 188, Tween 20 und Tween 80 gewählt wird, zu verbessern.

10. Arzneimittel nach einem der vorhergehenden Ansprüche, umfassend ein GLP-1-Derivat, wobei der lipophile Substituent 4 bis 40 Kohlenstoffatome, vorzugsweise 8 bis 25 Kohlenstoffatome, umfasst.

11. Arzneimittel nach einem der vorhergehenden Ansprüche, umfassend ein GLP-1-Derivat, wobei ein lipophiler Substituent an einem Aminosäurerest so angehängt ist, dass der lipophile Substituent eine Amidbindung mit einer Aminogruppe des Aminosäurerests bildet.

12. Arzneimittel nach einem der Ansprüche 1 bis 11, umfassend ein GLP-1-Derivat, wobei ein lipophiler Substituent an einem Aminosäurerest so angehängt ist, dass eine Aminogruppe des lipophilen Substituenten eine Amidbindung mit einer Carboxylgruppe des Aminosäurerests bildet.

13. Arzneimittel nach einem der vorhergehenden Ansprüche, umfassend ein GLP-1-Derivat, wobei der lipophile Substituent am Stammpeptid mittels eines Spacers angehängt ist.

14. Arzneimittel nach Anspruch 13, wobei der Spacer eine nicht-verzweigte Alkan-α,ω-Dicarbonsäuregruppe mit 1 bis 7 Methylengruppen ist, vorzugsweise zwei Methylengruppen, die eine Brücke zwischen einer Aminogruppe des Stammpeptids und einer Aminogruppe des lipophilen Substituenten bildet.

15. Arzneimittel nach Anspruch 13, wobei der Spacer ein Aminosäurerest außer Cys oder ein Dipeptid, wie Gly-Lys, oder eine nicht-verzweigte Alkan-α,ω-Aminosäure mit 1 bis 7 Methylengruppen ist, vorzugsweise 2 bis 4 Methylengruppen, die eine Brücke zwischen einer Aminogruppe des Stammpeptids und einer Aminogruppe des lipophilen Substituenten bildet.

16. Arzneimittel nach einem der vorhergehenden Ansprüche umfassend ein GLP-1-Derivat, wobei der lipophile Substituent ein teilweise oder komplett hydriertes Cyclopentanophenathren-Skelett umfasst.

17. Arzneimittel nach einem der Ansprüche 3 bis 15, wobei der lipophile Substituent eine geradkettige oder verzweigte Alkylgruppe ist.

18. Arzneimittel nach einem der Ansprüche 3 bis 15, umfassend ein GLP-1-Derivat, wobei der lipophile Substituent die Acylgruppe einer geradkettigen oder verzweigten Fettsäure ist.

19. Arzneimittel nach Anspruch 18, wobei die Acylgruppe aus der Gruppe, bestehend aus CH₃(CH₂)ₙCO-, wobei n 4 bis 38 ist, vorzugsweise CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- und CH₃(CH₂)₂₂CO-, gewählt wird.

20. Arzneimittel nach einem der Ansprüche 3 bis 15, umfassend ein GLP-1-Derivat, wobei der lipophile Substituent eine Acylgruppe einer geradkettigen oder verzweigten Alkan-α,ω-Dicarbonsäure ist.

21. Arzneimittel nach Anspruch 20, wobei die Acylgruppe aus der Gruppe, bestehend aus HOOC(CH₂)ₘCO-, wobei m 4 bis 38 ist, vorzugsweise 4 bis 24, gewählt wird, stärker bevorzugt aus der Gruppe, umfassend HOOC(CH₂)₁₄CO-, HOOC(CH₂)₁₆CO-, HOOC(CH₂)₁₈CO-, HOOC(CH₂)₂₀CO- und HOOC(CH₂)₂₂CO-.

22. Arzneimittel nach einem der Ansprüche 3 bis 15, umfassend ein GLP-1-Derivat, wobei der lipophile Substituent eine Gruppe der Formel CH₃(CH₂)ₚ((CH₂)_{q}COOH)CHNH-CO(CH₂)₂CO- ist, und wobei p und q ganze Zahlen sind, und p+q eine ganze Zahl von 8 bis 33, vorzugsweise von 12 bis 28, ist.

23. Arzneimittel nach einem der Ansprüche 3 bis 15, umfassend ein GLP-1-Derivat, wobei der lipophile Substituent eine Gruppe der Formel CH₃(CH₂)ᵣCO-NHCH(COOH)(CH₂)₂CO- ist, und wobei r eine ganze Zahl von 10 bis 24 ist.

24. Arzneimittel nach einem der Ansprüche 3 bis 15, umfassend ein GLP-1-Derivat, wobei der lipophile Substituent eine Gruppe der Formel CH₃(CH₂)ₛCO-NHCH((CH₂)₂COOH)CO- ist, und wobei s eine ganze Zahl von 8 bis 24 ist.

25. Arzneimittel nach einem der Ansprüche 3 bis 15, umfassend ein GLP-1-Derivat, wobei der lipophile Substituent eine Gruppe der Formel - NHCH(COOH)(CH₂)₄NH-CO(CH₂)ᵤCH₃ ist, und wobei u eine ganze Zahl von 8 bis 18 ist.

26. Arzneimittel nach einem der Ansprüche 3 bis 15, umfassend ein GLP-1-Derivat, wobei der lipophile Substituent eine Gruppe der Formel -NHCH(COOH)(CH₂)₄NH-COCH((CH₂)₂COOH)NH-CO(CH₂)_{w}CH₃ ist, und wobei w eine ganze Zahl von 10 bis 16 ist.

27. Arzneimittel nach einem der Ansprüche 3 bis 15, umfassend ein GLP-1-Derivat, wobei der lipophile Substituent eine Gruppe der Formel -NHCH(COOH)(CH₂)₄NH-CO(CH₂)₂CH(COCH)NH-CO(CH₂)ₓCH₃ ist, und wobei x eine ganze Zahl von 10 bis 16 ist.

28. Arzneimittel nach einem der Ansprüche 3 bis 15, umfassend ein GLP-1-Derivat, wobei der lipophile Substituent eine Gruppe der Formel -NHCH(COOH)(CH₂)₄NH-CO((CH₂)₂CH(COOH)NH-CO(CH₂)_{y}CH₃ ist, und wobei y Null oder eine ganze Zahl von 1 bis 22 ist.

29. Arzneimittel nach einem der vorhergehenden Ansprüche, umfassend ein GLP-1-Derivat, wobei das Stammpeptid GLP-1(A-B), und wobei A eine ganze Zahl von 1 bis 7 und B eine ganze Zahl von 38 bis 45 ist, oder ein Analogon davon ist.

30. Arzneimittel nach Anspruch 29, wobei das Stammpeptid aus der Gruppe, bestehend aus GLP-1(7-35); GLP-1(7-36); GLP-1(7-36)-Amid; GLP-1(7-37); GLP-1(7-38); GLP-1(7-39); GLP-1(7-40) und GLP-1(7-41) und Analoga davon gewählt wird.

31. Arzneimittel nach Anspruch 29, wobei das Stammpeptid aus der Gruppe, bestehend aus GLP-1(1-35); GLP-1(1-36); GLP-1(1-36)-Amid; GLP-1(1-37); GLP-1(1-38); GLP-1(1-39); GLP-1(1-40) und GLP-1(1-41) und Analoga davon gewählt wird.

32. Arzneimittel nach einem der vorhergehenden Ansprüche, umfassend ein GLP-1-Derivat, wobei insgesamt bis 15, vorzugsweise bis 10, stärker bevorzugt bis 6, Aminosäurereste mit einem α-Aminosäurerest, der mit dem genetischen Code kodiert werden kann, ausgetauscht ist.

33. Arzneimittel nach einem der vorhergehenden Ansprüche, umfassend ein GLP-1-Derivat, wobei das Stammpeptid aus der Gruppe, bestehend aus Arg²⁶-GLP-1(7-37); Arg³⁴-GLP-1(7-37); Lys³⁸-GLP-1(7-37); Arg^{26,34}Lys³⁸-GLP-1(7-37); Arg^{26,34}Lys³⁸-GLP-1(7-38); Arg^{26,34}Lys³⁸-GLP-1(7-39); Arg^{26,34}Lys⁴⁰-GLP-1(7-40); Arg²⁶Lys³⁸-GLP-1(7-37); Arg³⁴Lys³⁸-GLP-1(7-37); Arg²⁶Lys³⁸-GLP-1(7-39); Arg³⁴Lys⁴⁰-GLP-1(7-40); Arg^{26,34}Lys^{38,39}-GLP-1(7-39); Arg^{26,34}Lys^{38,40}-GLP-1(7-40); Gly⁸Arg²⁶-GLP-1(7-37); Gly⁸Arg³⁴-GLP-1(7-37); Gly⁸Lys³⁸-GLP-1(7-37); Gly⁸Arg^{26,34}Lys³⁸-GLP-1(7-37); Gly⁸Arg^{26,34}Lys³⁸-GLP-1(7-39); Gly⁸Arg^{26,34}Lys⁴⁰-GLP-1(7-40); Gly⁸Arg²⁶Lys³⁸-GLP-1(7-37); Gly⁸Arg³⁴Lys³⁸-GLP-1(7-37); Gly⁸Arg²⁶Lys³⁹-GLP-1(7-39); Gly⁸Arg³⁴Lys⁴⁰-GLP-1(7-40); Gly⁸Arg^{26,34}Lys^{38,39}-GLP-1(7-39) und Gly⁸Arg^{26,34}Lys^{38,40}-GLP-1(7-40) gewählt wird.

34. Arzneimittel nach einem der Ansprüche 1 bis 32, umfassend ein GLP-1-Derivat, wobei das Stammpeptid aus der Gruppe, bestehend aus Arg^{26,34}Lys³⁸GLP-1(7-38); Arg^{26,34}Lys³⁹GLP-1(7-39); Arg^{26,34}Lys⁴⁰GLP-1(7-40); Arg^{26,34}Lys⁴¹GLP-1(7-41); Arg^{26,34}Lys⁴²GLP-1(7-42); Arg^{26,34}Lys⁴³GLP-1(7-43); Arg^{26,34}Lys⁴⁴GLP-1(7-44); Arg^{26,34}Lys⁴⁵GLP-1(7-45); Arg^{26,34}Lys³⁸GLP-1(1-38); Arg^{26,34}Lys³⁹GLP-1(1-39); Arg^{26,34}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1(1-44); Arg^{26,34}Lys⁴⁵GLP-1(1-45); Arg^{26,34}Lys³⁸GLP-1(2-38); Arg^{26,34}Lys³⁹GLP-1(2-39); Arg^{26,34}Lys⁴⁰GLP-1(2-40); Arg^{26,34}Lys⁴¹GLP-1(2-41); Arg^{26,34}Lys⁴²GLP-1(2-42); Arg^{26,34}Lys⁴³GLP-1(2-43); Arg^{26,34}Lys⁴⁴GLP-1(2-44); Arg^{26,34}Lys⁴⁵GLP-1(2-45); Arg^{26,34}Lys³⁸GLP-1(3-38); Arg^{26,34}Lys³⁹GLP-1(3-39); Arg^{26,34}Lys⁴⁰GLP-1(3-40); Arg^{26,34}Lys⁴¹GLP-1(3-41); Arg^{26,34}Lys⁴²GLP-1(3-42); Arg^{26,34}Lys⁴³GLP-1(3-43); Arg^{26,34}Lys⁴⁴GLP-1(3-44); Arg^{26,34}Lys⁴⁵GLP-1(3-45); Arg^{26,34}Lys³⁸GLP-1(4-38); Arg^{26,34}Lys³⁹GLP-1(4-39); Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,34}Lys⁴⁵GLP-1(4-45); Arg^{26,34}Lys³⁸GLP-1(5-38); Arg^{26,34}Lys³⁹GLP-1(5-39); Arg^{26,34}Lys⁴⁰GLP-1(5-40); Arg^{26,34}Lys⁴¹GLP-1(5-41); Arg^{26,34}Lys⁴²GLP-1(5-42); Arg^{26,34}Lys⁴³GLP-1(5-43); Arg^{26,34}Lys⁴⁴GLP-1(5-44); Arg^{26,34}Lys⁴⁵GLP-1(5-45); Arg^{26,34}Lys³⁸GLP-1(6-38); Arg^{26,34}Lys³⁹GLP-1(6-39); Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁸GLP-1(1-38); Arg³⁴Lys³⁸GLP-1(1-38); Arg^{26,34}Lys^{36,38}GLP-1(1-38); Arg²⁶Lys³⁸GLP-1(7-38); Arg^{26,34}Lys^{36,38}GLP-1(7-38); Arg^{26,34}Lys³⁸GLP-1(7-38); Arg²⁶Lys³⁸GLP-1(1-39); Arg³⁴Lys³⁹-GLP-1(1-39); Arg^{26,34}Lys^{38,39}GLP-1(1-39); Arg²⁶Lys³⁹GLP-1(7-39); Arg³⁴Lys³⁸GLP-1(7-39) und Arg^{26,34}Lys^{38,39}GLP-1(7-39) gewählt wird.

35. Verfahren zur Verbesserung der Löslichkeit und/oder Stabilität von GLP-1, eines Fragments oder eines Analogons davon, **dadurch gekennzeichnet, dass** ein lipophiler Substituent in einen der Aminosäurereste des Stammpeptids eingeführt wird.

36. Verfahren nach Anspruch 35, wobei ein lipophiler Substituent in einen der Aminosäurereste in Position 18-38, vorzugsweise 26-34, eingeführt wird.

37. Verfahren nach Anspruch 35 oder 36, wobei der lipophile Substituent 4 bis 40 Kohlenstoffatome, vorzugsweise 8 bis 25 Kohlenstoffatome, umfasst.

38. Verfahren nach einem der Ansprüche 35 bis 37, wobei der lipophile Substituent die Acylgruppe einer geradekettigen oder verzweigten Fettsäure ist.

39. Verfahren nach Anspruch 36, wobei die Acylgruppe aus der Gruppe, bestehend aus CH₃(CH₂)ₙCO-, und wobei n 4 bis 38 ist, vorzugsweise CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- und CH₃(CH₂)₂₂CO-, gewählt wird.

40. Verfahren nach einem der Ansprüche 35 bis 39, wobei das GLP-1 GLP-1(A-B) , und wobei A eine ganze Zahl von 1 bis 7 und B eine ganze Zahl von 38 bis 45 ist, oder ein Analogon davon ist.

41. Verfahren nach Anspruch 40, wobei das GLP-1 aus der Gruppe, bestehend aus GLP-1(7-35); GLP-1(7-36); GLP-1(7-36)-Amid; GLP-1(7-37); GLP-1(7-38); GLP-1(7-39); GLP-1(7-40); GLP-1(7-41); und Analoga davon gewählt wird.

42. Verfahren nach Anspruch 40, wobei das GLP-1 aus der Gruppe, bestehend aus GLP-1(1-35); GLP-1(1-36); GLP-1(1-36)-Amid; GLP-1(1-37); GLP-1(1-38); GLP-1(1-39); GLP-1(1-40); GLP-1(1-41); und Analoga davon gewählt wird.

## Revendications

1. Composition pharmaceutique comportant un dérivé de GLP-1 qui a une structure en hélice comme mesuré par CD à 222 nm dans H₂O à 22 ± 2°C, dépassant 25 %, de préférence dans la plage allant de 25 % à 50 %, à une concentration peptidique d'environ 10 µM, la concentration du dérivé de GLP-1 n'étant pas inférieure à 0,5 mg/ml, et un conservateur.

2. Composition pharmaceutique selon la revendication 1, dans laquelle la concentration en dérivé de GLP-1 n'est pas inférieure à 5 mg/ml, de manière plus préférée pas inférieure à 10 mg/ml et, de préférence, pas supérieure à 100 mg/ml.

3. Composition pharmaceutique selon la revendication 1 ou 2, comportant un dérivé de GLP-1 dans lequel au moins un résidu d'acide aminé du peptide parent a un substituant lipophile attaché.

4. Composition pharmaceutique selon la revendication 3, comportant un dérivé de GLP-1 ayant un substituant lipophile qui est attaché à l'un quelconque des résidus d'acide aminé aux positions 18-38, de préférence 26-34.

5. Composition pharmaceutique selon l'une quelconque des revendications précédentes, comportant de plus un véhicule ou support pharmaceutiquement acceptable.

6. Composition pharmaceutique selon l'une quelconque des revendications précédentes, comportant de plus un agent isotonique, de préférence sélectionné parmi le groupe constitué de chlorure de sodium, mannitol et glycérol.

7. Composition pharmaceutique selon l'une quelconque des revendications précédentes, dans laquelle le conservateur est sélectionné parmi le groupe constitué de phénol, m-crésol, p-hydroxybenzoate de méthyle, p-hydroxybenzoate de butyle et alcool benzylique.

8. Composition pharmaceutique selon l'une quelconque des revendications précédentes, comportant de plus un tampon, de préférence sélectionné parmi le groupe constitué d'acétate de sodium, citrate, glycylglycine, histidine, 2-phényléthanol et phosphate de sodium.

9. Composition pharmaceutique selon l'une quelconque des revendications précédentes, comportant de plus un tensioactif capable d'améliorer la solubilité et/ou la stabilité du dérivé de GLP-1, de préférence sélectionné parmi le poloxymère 188, tween 20 et tween 80.

10. Composition pharmaceutique selon l'une quelconque des revendications précédentes, comportant un dérivé de GLP-1 dans lequel le substituant lipophile comporte de 4 à 40 atomes de carbone, de préférence de 8 à 25 atomes de carbone.

11. Composition pharmaceutique selon l'une quelconque des revendications précédentes, comportant un dérivé de GLP-1 dans lequel un substituant lipophile est attaché à un résidu d'acide aminé de telle sorte qu'un groupe carboxyle du substituant lipophile forme une liaison amide avec un groupe amino du résidu d'acide aminé.

12. Composition pharmaceutique selon l'une quelconque des revendications 1 à 11, comportant un dérivé de GLP-1 dans lequel un substituant lipophile est attaché à un résidu d'acide aminé de telle sorte qu'un groupe amino du substituant lipophile forme une liaison amide avec un groupe carboxyle du résidu d'acide aminé.

13. Composition pharmaceutique selon l'une quelconque des revendications précédentes, comportant un dérivé de GLP-1 dans lequel le substituant lipophile est attaché au peptide parent par l'intermédiaire d'un élément d'espacement.

14. Composition pharmaceutique selon la revendication 13, dans laquelle l'élément d'espacement est un groupe d'acide α,ω-dicarboxylique d'alcane non-ramifié ayant de 1 à 7 groupes méthylène, de préférence deux groupes méthylène, qui forment un pont entre un groupe amino du peptide parent et un groupe amino du substituant lipophile.

15. Composition pharmaceutique selon la revendication 13, dans laquelle l'élément d'espacement est un résidu d'acide aminé à l'exception de Cys, ou un dipeptide tel que Gly-Lys ou tout acide α,ω-dicarboxylique d'alcane non-ramifié ayant de 1 à 7 groupes méthylène, de préférence 2 à 4 groupes méthylène, qui forment un pont entre un groupe amino du peptide parent et un groupe amino du substituant lipophile.

16. Composition pharmaceutique selon l'une quelconque des revendications précédentes, comportant un dérivé de GLP-1 dans lequel le substituant lipophile comporte un squelette de cyclopentanophénathrène partiellement ou complètement hydrogéné.

17. Composition pharmaceutique selon l'une quelconque des revendications 3 à 15, dans laquelle le substituant lipophile est un groupe alkyle à chaîne rectiligne ou ramifié.

18. Composition pharmaceutique selon l'une quelconque des revendications 3 à 15, comportant un dérivé de GLP-1 dans lequel le substituant lipophile est le groupe acyle d'un acide gras à chaîne rectiligne ou ramifié.

19. Composition pharmaceutique selon la revendication 18, dans laquelle le groupe acyle est sélectionné parmi le groupe comportant CH₃(CH₂)ₙCO-, où n est compris entre 4 et 38, de préférence CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- et CH₃(CH₂)₂₂CO-.

20. Composition pharmaceutique selon l'une quelconque des revendications 3 à 15, comportant un dérivé de GLP-1 dans lequel le substituant lipophile est un groupe acyle d'un acide α,ω-dicarboxylique d'alcane à chaîne rectiligne ou ramifié.

21. Composition pharmaceutique selon la revendication 20, dans laquelle le groupe acyle est sélectionné parmi le groupe comportant HOOC(CH₂)ₘCO-, où m est compris entre 4 et 38, de préférence entre 4 et 24, sélectionné de manière plus préférée parmi le groupe comportant HOOC(CH₂)₁₄CO-, HOOC(CH₂)₁₆CO-, HOOC(CH₂)₁₈CO-, HOOC(CH₂)₂₀CO- et HOOC(CH₂)₂₂CO-.

22. Composition pharmaceutique selon l'une quelconque des revendications 3 à 15, comportant un dérivé de GLP-1 dans lequel le substituant lipophile est un groupe ayant la formule suivante CH₃(CH2)ₚ((CH₂)_{q}COOH)CHNH-CO(CH₂)₂CO-, où p et q sont des nombres entiers et p+q est un nombre entier compris entre 8 et 33, de préférence entre 12 et 28.

23. Composition pharmaceutique selon l'une quelconque des revendications 3 à 15, comportant un dérivé de GLP-1, dans lequel le substituant lipophile est un groupe ayant la formule CH₃(CH₂)ᵣCO-NHCH(COOH) (CH₂)₂CO-, où r est un nombre entier compris entre 10 et 24.

24. Composition pharmaceutique selon l'une quelconque des revendications 3 à 15, comportant un dérivé de GLP-1, dans lequel le substituant lipophile est un groupe ayant la formule CH₃(CH₂)ₛCO-NHCH ((CH₂)₂COOH) CO-, où s est un nombre entier compris entre 8 et 24.

25. Composition pharmaceutique selon l'une quelconque des revendications 3 à 15, comportant un dérivé de GLP-1, dans lequel le substituant lipophile est un groupe ayant la formule -NHCH(COOH)(CH₂)₄NH-CO(CH₂)ᵤCH₃, où u est un nombre entier compris entre 8 et 18.

26. Composition pharmaceutique selon l'une quelconque des revendications 3 à 15, comportant un dérivé de GLP-1, dans lequel le substituant lipophile est un groupe ayant la formule -NHCH(COOH) (CH₂)₄NH-COCH((CH₂)₂COOH)NH-CO(CH₂)_{w}CH₃, où w est un nombre entier compris entre 10 et 16.

27. Composition pharmaceutique selon l'une quelconque des revendications 3 à 15, comportant un dérivé de GLP-1, dans lequel le substituant lipophile est un groupe ayant la formule -NHCH(COOH) (CH₂)₄NH-CO(CH₂)₂CH(COOH)NH-CO(CH₂)ₓCH₃, où x est un nombre entier compris entre 10 et 16.

28. Composition pharmaceutique selon l'une quelconque des revendications 3 à 15, comportant un dérivé de GLP-1, dans lequel le substituant lipophile est un groupe ayant la formule -NHCH(COOH) (CH₂)₄NH-CO(CH₂)₂CH(COOH)NH-CO(CH₂)_{y}CH₃, où y est égal à zéro ou est un nombre entier compris entre 1 et 22.

29. Composition pharmaceutique selon l'une quelconque des revendications précédentes, comportant un dérivé de GLP-1 dans lequel le peptide parent est GLP-1(A-B), où A est un nombre entier compris entre 1 et 7 et B est un nombre entier compris entre 38 et 45, ou un analogue de celui-ci.

30. Composition pharmaceutique selon la revendication 29, dans laquelle le peptide parent est sélectionné parmi le groupe comportant GLP-1 (7-35), GLP-1 (7-36), GLP-1 (7-36) amide, GLP-1 (7-37), GLP-1 (7-38) , GLP-1 (7-39), GLP-1 (7-40) et GLP-1 (7-41), et des analogues de ceux-ci.

31. Composition pharmaceutique selon la revendication 29, dans laquelle le peptide parent est sélectionné parmi le groupe comportant GLP-1(1-35), GLP-1(1-36), GLP-1(1-36)amide, GLP-1(1-37), GLP-1(1-38), GLP-1(1-39), GLP-1(1-40), GLP-1(1-41), et des analogues de ceux-ci.

32. Composition pharmaceutique selon l'une quelconque des revendications précédentes, comportant un dérivé de GLP-1 dans lequel un total allant jusqu'à quinze, de préférence jusqu'à dix, de manière plus préférée jusqu'à six, résidus d'acide aminé a été échangé avec un quelconque résidu d'acide α-aminé qui peut être codé par le code génétique.

33. Composition pharmaceutique selon l'une quelconque des revendications précédentes, comportant un dérivé de GLP-1 dans lequel le peptide parent est sélectionné parmi le groupe comportant Arg²⁸-GLP-1(7-37), Arg³⁴-GLP-1(7-37), Lys³⁶-GLP-1(7-37), Arg^{26,34}Lys³⁶-GLP-1(7-37), Arg^{26,34}Lys³⁸GLP-1(7-38), Arg^{26,34}Lys³⁹-GLP-1(7-39), Arg^{26,34}Lys⁴⁰-GLP-1(7-40), Arg²⁶Lys³⁶-GLP-1(7-37), Arg³⁴Lys³⁶-GLP-1(7-37), Arg²⁶Lys³⁹-GLP-1(7-39), Arg³⁴Lys⁴⁰-GLP-1(7-40), Arg^{26,34}Lys^{36,39}-GLP-1(7-39), Arg^{26,34}Lys^{36,40}-GLP-1(7-40), Gly⁸Arg²⁶-GLP-1(7-37), Gly⁸Arg³⁴-GLP-1(7-37), Gly⁸Lys³⁶-GLP-1(7-37), Gly⁸Arg^{26,34}Lys³⁶-GLP-1(7-37), Gly⁸Arg^{26,34}Lys³⁹-GLP-1(7-39), Gly⁸Arg^{26,34}Lys⁴⁰-GLP-1(7-40), Gly⁸Arg²⁶Lys³⁶-GLP-1(7-37), Gly⁸Arg³⁴Lys³⁶-GLP-1(7-37), Gly⁸Arg²⁶Lys³⁹-GLP-1(7-39), Gly⁸Arg³⁴Lys⁴⁰-GLP-1(7-40), Gly⁸Arg^{26,34}Lys^{36,39}-GLP-1(7-39) et Gly⁸Arg^{26,34}Lys^{36,40}-GLP-1(7-40).

34. Composition pharmaceutique selon l'une quelconque des revendications 1 à 32, comportant un dérivé de GLP-1, dans lequel le peptide parent est sélectionné parmi le groupe comportant Arg^{26,34}Lys³⁸-GLP-1(7-38), Arg^{26,34}Lys³⁹-GLP-1(7-39), Arg^{26,34}Lys⁴⁰-GLP-1(7-40), Arg^{26,34}Lys⁴¹-GLP-1(7-41), Arg^{26,34}Lys⁴²-GLP-1(7-42), Arg^{26,34}Lys⁴³-GLP-1(7-43), Arg^{26,34}Lys⁴⁴-GLP-1(7-44), Arg^{26,34}Lys⁴⁵GLP-1(7-45), Arg^{26,34}Lys³⁸GLP-1(1-38), Arg^{26,34}Lys³⁹GLP-1(1-39), Arg^{26,34}Lys⁴⁰GLP-1(1-40), Arg^{26,34}Lys⁴¹GLP-1(1-41), Arg^{26,34}Lys⁴²GLP-1(1-42), Arg^{26,34}Lys⁴³GLP-1(1-43), Arg^{26,34}Lys⁴⁴GLP-1(1-44), Arg^{26,34}Lys⁴⁵GLP-1(1-45), Arg^{26,34}Lys³⁸GLP-1(2-38), Arg^{26,34}Lys³⁹GLP-1(2-39), Arg^{26,34}Lys⁴⁰GLP-1(2-4 0) , Arg^{26,34}Lys⁴¹GLP-1(2-41), Arg^{26,34}Lys⁴²GLP-1(2-42), Arg^{26,34}Lys⁴³GLP-1(2-43), Arg^{26,34}Lys⁴⁴GLP-1(2-44), Arg^{26,34}Lys⁴⁵GLP-1(2-45), Arg^{26,34}Lys³⁸GLP-1(3-38), Arg^{26,34}Lys³⁹GLP-1(3-39), Arg^{26,34}Lys⁴⁰GLP-1(3-40), Arg^{26,34}Lys⁴¹GLP-1(3-41), Arg^{26,34}Lys⁴²GLP-1(3-42), Arg^{26,34}Lys⁴³GLP-1(3-43), Arg^{26,34}Lys⁴⁴GLP-1(3-44), Arg^{26,34}Lys⁴⁵GLP-1(3-45), Arg^{26,34}Lys³⁸GLP-1(4-38), Arg^{26,34}Lys³⁹GLP-1(4-39), Arg^{26,34}Lys⁴⁰GLP-1(4-40), Arg^{26,34}Lys⁴¹GLP-1(4-41), Arg^{26,34}Lys⁴²GLP-1(4-42), Arg^{26,34}Lys⁴³GLP-1(4-43), Arg^{26,34}Lys⁴⁴GLP-1(4-44), Arg^{26,34}Lys⁴⁵GLP-1(4-45), Arg^{26,34}Lys³⁸GLP-1(5-38), Arg^{26,34}Lys³⁹GLP-1(5-39), Arg^{26,34}Lys⁴⁰GLP-1(5-40), Arg^{26,34}Lys⁴¹GLP-1(5-41), Arg^{26,34}Lys⁴²GLP-1(5-42), Arg^{26,34}Lys⁴³GLP-1(5-43), Arg^{26,34}Lys⁴⁴GLP-1(5-44), Arg^{26,34}Lys⁴⁵GLP-1(5-45), Arg^{26,34}Lys³⁸GLP-1(6-38), Arg^{26,34}Lys³⁹GLP-1(6-39), Arg^{26,34}Lys⁴⁰GLP-1(6-40), Arg^{26,34}Lys⁴¹GLP-1(6-41), Arg^{26,34}Lys⁴²GLP-1(6-42), Arg^{26,34}Lys⁴³GLP-1(6-43), Arg^{26,34}Lys⁴⁴GLP-1(6-44), Arg^{26,34}Lys⁴⁵GLP-1(6-45), Arg²⁶Lys³⁸GLP-1(1-38), Arg³⁴Lys³⁸GLP-1(1-38), Arg^{26,34}Lys^{36,38}GLP-1(1-38), Arg²⁶Lys³⁸GLP-1(7-38), Arg³⁴Lys³⁸GLP-1(7-38), Arg^{26,34}Lys^{36,38}GLP-1(7-38), Arg^{26,34}Lys³⁸GLP-1(7-38), Arg²⁶Lys³⁹GLP-1(1-39), Arg³⁴Lys³⁹GLP-1(1-39), Arg^{26,34}Lys^{36,39}GLP-1(1-39), Arg²⁶Lys³⁹GLP-1(7-39), Arg³⁴Lys³⁹GLP-1(7-39), et Arg^{26,34}Lys^{36,39}GLP-1(7-39).

35. Procédé pour améliorer la solubilité et/ou stabilité de GLP-1 ou d'un fragment ou analogue de celui-ci, **caractérisé en ce qu'**un substituant lipophile est introduit sur l'une quelconque des résidus d'acide aminé du peptide parent.

36. Procédé selon la revendication 35, dans lequel un substituant lipophile est introduit sur l'un quelconque des résidus d'acide aminé aux positions 18-38, de préférence 26-34.

37. Procédé selon la revendication 35 ou 36, dans lequel le substituant lipophile comporte de 4 à 40 atomes de carbone, de préférence de 8 à 25 atomes de carbone.

38. Procédé selon l'une quelconque des revendications 35 à 37, dans lequel le substituant lipophile est le groupe acyle d'un acide gras à chaîne rectiligne ou ramifié.

39. Procédé selon la revendication 36, dans lequel le groupe acyle est sélectionné parmi le groupe comportant CH₃(CH₂)ₙCO-, où n est compris entre 4 et 38, de préférence CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- et CH₃(CH₂)₂₂CO-.

40. Procédé selon l'une quelconque des revendications 35 à 39, dans lequel le GLP-1 est GLP-1(A-B), où A est un nombre entier compris entre 1 et 7 et B est un nombre entier compris entre 38 et 45, ou un analogue de celui-ci.

41. Procédé selon la revendication 40, dans lequel le GLP-1 est sélectionné parmi le groupe comportant GLP-1 (7-35), GLP-1(7-36), GLP-1 (7-36) amide, GLP-1(7-37), GLP-1(7-38), GLP-1(7-39), GLP-1 (7-40) et GLP-1(7-41), et des analogues de ceux-ci.

42. Procédé selon la revendication 40, dans lequel le GLP-1 est sélectionné parmi le groupe comportant GLP-1 (1-35), GLP-1 (1-36), GLP-1 (1-36) amide, GLP-1 (1-37), GLP-1 (1-38), GLP-1 (1-39), GLP-1 (1-40), GLP-1(1-41), et des analogues de ceux-ci.
